# EUROPEAN PATENT APPLICATION

(11) **EP 4 759 926 A1**
(43) Date of publication of application: **17.06.2026**
(21) Application number: 24851982.9
(22) Date of filing: 09.08.2024
(51) Int. Cl.: C12N 5/22, C12N 1/00, C12N 5/10, C12N 5/077, C12N 5/079, C12N 7/00, C12Q 1/02

(54) **PLURIPOTENT STEM CELL HAVING DOUBLED CHROMOSOME**

(30) Priority: 10.08.2023 JP 2023131333
(71) Applicant: Tokyo Metropolitan Institute of Medical Science, Tokyo 156-8506 (JP)
(72) Inventor: MIYAOKA Yuichiro, Tokyo 156-8506 (JP); NAKAJIMA Ittetsu, Tokyo 156-8506 (JP)
(74) Representative: J A Kemp LLP
(86) International application number: PCT/JP2024/080141
(87) International publication number: WO 2025/033565

(57) **Abstract**

Provided is a (N₁+N₂)-ploid pluripotent stem cell comprising a fused cell of a N₁-ploid (wherein N₁ represents an integer of 2 to 16) pluripotent stem cell and a N₂-ploid (wherein N₂ represents an integer of 2 to 16) pluripotent stem cell.

## Description

### [TECHNICAL FIELD]

The present invention relates to a pluripotent stem cell having doubled chromosomes.

### [BACKGROUND ART]

Human pluripotent stem cells, which can differentiate into various cell types in the body, contribute greatly not only to basic research but also to drug discovery through the generation of disease models and to medical applications through cell transplantation therapy. Compared to ES cells established by destroying human fertilized eggs (Non-patent literature 1) (1), iPS cells established by introducing Yamanaka factors into differentiated cells collected from humans raise fewer ethical issues (Non-patent literature 2) (2). In addition, since the cells can be established using somatic cells from an individual in need of the cells, they are expected to be applicable to tailor-made medicine, such as transplantation using the patient's own cells. Thus, human pluripotent stem cells, particularly human iPS cells, greatly contribute to the development of regenerative medicine.

However, there are also problems with pluripotent stem cells, which need to be solved. One of the most significant problems is that the maturity of differentiation-induced cells is generally low in all cell types. For example, in the case of cardiomyocyte differentiation, the cardiomyocytes are known to differentiate only to the level of fetal cardiomyocytes. Therefore, in order to increase the maturity of pluripotent stem cell-derived cardiomyocytes, a number of techniques have been used, including prolonged culture time, addition of a low-molecular-weight compound, addition of a hormone such as glucocorticoid or T3, regulation of an energy source substance such as a carbohydrate or a lipid, regulation of interaction with an intercellular/extracellular matrix, physical/electrical stimulation, *in vivo* transplantation into mice, and three-dimensional culture (Non-patent literature 3) (3). However, none of these techniques has become a definitive technique.

Some of the differentiated and matured cells that make up our bodies have been polyploidized. This polyploidization is one indicator of differentiation and maturation. For example, among the cardiomyocytes that make up the adult human heart, only 10% or less remain diploid, and about 80% have been polyploidized (Non-patent literature 4) (4). In addition, about half of the hepatocytes have been polyploidized (Non-patent literature 5) (5). Polyploidization has also been observed in other cells, such as skeletal myocytes, placental trophoblast giant cells, megakaryocytes, and osteoclasts (Non-patent literature 6) (6). Such polyploidization is one of the processes of cell differentiation but is very difficult to reproduce by inducing differentiation of normal diploid iPS cells.

### [PRIOR ART LITERATURE]

### [Non-patent literature]

[Non-patent literature 1] Thomson JA, Itskovitz-Eldor J, Shapiro SS, Waknitz MA, Swiergiel JJ, Marshall VS, et al. Embryonic stem cell lines derived from human blastocysts. Science. 1998 Nov 6; 282(5391): 1145-7.
[Non-patent literature 2] Takahashi K, Tanabe K, Ohnuki M, Narita M, Ichisaka T, Tomoda K, et al. Induction of pluripotent stem cells from adult human fibroblasts by defined factors. Cell. 2007 Nov 30; 131(5): 861-72.
[Non-patent literature 3] Ahmed RE, Anzai T, Chanthra N, Uosaki H. A Brief Review of Current Maturation Methods for Human Induced Pluripotent Stem Cells-Derived Cardiomyocytes. Front Cell Dev Biol. 2020 Mar 19; 8: 178.
[Non-patent literature 4] Derks W, Bergmann O. Polyploidy in cardiomyocytes: roadblock to heart regeneration? Circ Res. 2020 Feb 14; 126(4):552-65.
[Non-patent literature 5] Sladky VC, Eichin F, Reiberger T, Villunger A. Polyploidy control in hepatic health and disease. J Hepatol. 2021 Nov; 75(5): 1177-91.
[Non-patent literature 6] Fang J, de Bruin A, Villunger A, Schiffelers R, Lei Z, Sluijter JPG. Cellular polyploidy in organ homeostasis and regeneration. Protein Cell. 2022 Dec 31

### [SUMMARY OF INVENTION]

### [Problem to be Solved by the Invention]

As described above, there is a demand for a method for easily polyploidizing diploid pluripotent stem cells.

### [Means for Solving the Problem]

After diligent study to solve the above problem, the present inventors succeeded in fusing pluripotent stem cells having multipotency with each other to polyploidize them in an undifferentiated state, thereby completing the present invention.

Thus, the present invention is as follows.
[1] A (N₁ + N₂)-ploid pluripotent stem cell comprising a fused cell of a N₁-ploid (wherein N₁ represents an integer from 2 to 16) pluripotent stem cell and a N₂-ploid (wherein N₂ represents an integer from 2 to 16) pluripotent stem cell.
[2] The pluripotent stem cell according to [1], wherein N₁ and N₂ are 2 or 4.
[3] The pluripotent stem cell according to [1], wherein the pluripotent stem cell is an iPS cell.
[4] A method for producing a (N₁ + N₂)-ploid pluripotent stem cell, comprising a step of fusing a N₁-ploid (wherein N₁ represents an integer from 2 to 16) pluripotent stem cell and a N₂-ploid (wherein N₂ represents an integer from 2 to 16) pluripotent stem cell by culturing them in the presence of a virus.
[5] The method according to [4], wherein N₁ and N₂ are 2 or 4.
[6] The method according to [4], wherein the pluripotent stem cell is an iPS cell.
[7] The method according to [4], wherein the virus is Sendai virus.
[8] A method for producing a cardiomyocyte, comprising a step of culturing the pluripotent stem cell according to any one of [1]-[3] in the presence of a GSK-3 inhibitor.
[9] A method for producing a hepatocyte, comprising the steps of: treating the pluripotent stem cell according to any one of [1]-[3] with Activin A in the presence of a GSK-3 inhibitor to induce it into an endodermal cell; treating the induced endodermal cell with a fibroblast growth factor and an osteogenic factor to induce it into a hepatic progenitor cell; and further treating the hepatic progenitor cell with a hepatocyte growth factor.
[10] A method for producing a cerebral organoid, comprising the steps of: culturing the pluripotent stem cell according to any one of [1]-[3] in the presence of a fibroblast growth factor and a ROCK inhibitor to induce it into an embryoid body; culturing the embryoid body in a medium containing N2 supplement to induce it into a neuroepithelial cell; and further culturing it in a medium containing N2 supplement and B27 supplement.
[11] A method for producing a cardiac organoid, comprising the following steps (a)-(d):
   (a) a step of culturing the cell according to any one of [1]-[3] on a low attachment plate to induce it into an embryoid body;
   (b) a step of treating the embryoid body obtained in step (a) with an GSK-3 inhibitor, an osteogenic factor, and Activin A to induce it into a mesoderm;
   (c) a step of further treating the mesoderm induced in step (b) with Wnt-C59 to induce it into a cardiac mesoderm; and
   (d) a step of treating the cardiac mesoderm induced in step (c) with a GSK-3 inhibitor.
[12] A method for producing a hepatic organoid, comprising a step of culturing the pluripotent stem cell according to any one of [1]-[3] together with an iPS cell-derived endodermal cell, a vascular endothelial cell, and a mesenchymal stem cell to allow self-aggregation.
[13] A method for producing a skeletal myocyte, comprising a step of culturing the pluripotent stem cell according to any one of [1]-[3] in the presence of a TGF-β receptor inhibitor.
[14] A method for testing cardiotoxicity of a test substance, comprising the steps of: contacting the test substance with the pluripotent stem cell according to any one of [1]-[3], a cardiomyocyte generated by the method according to [8], or a cardiac organoid generated by the method according to [11]; and evaluating the test substance as a cardiotoxic substance by using cell death, cell membrane potential, calcium transient, or reduced muscle contractility as an indicator.
[15] A method for screening a drug that improves cardiomyocyte function, comprising the steps of: contacting a test substance with the pluripotent stem cell according to any one of [1]-[3], a cardiomyocyte generated by the method according to [8], or a cardiac organoid generated by the method according to [11]; and selecting the test substance as a drug that improves cardiomyocyte function by using muscle contractility or beating rhythm as an indicator.
[16] A method for testing hepatotoxicity of a test substance, comprising the steps of: contacting the test substance with the pluripotent stem cell according to any one of [1]-[3], a hepatocyte generated by the method according to [9], or a hepatic organoid generated by the method according to [12]; and evaluating the test substance as a hepatotoxic substance by using cell death or mitochondrial toxicity as an indicator.
[17] A method for screening a drug that improves hepatocyte function, comprising the steps of: contacting a test substance with the pluripotent stem cell according to any one of [1]-[3], a hepatocyte generated by the method according to [9], or a hepatic organoid generated by the method according to [12]; and selecting the test substance as a drug that improves hepatocyte function by using drug-metabolizing ability as an indicator.
[18] A method for screening a drug that improves skeletal myocyte function, comprising the steps of: contacting a test substance with the pluripotent stem cell according to any one of [1]-[3] or a skeletal myocyte generated by the method according to [13]; and selecting the test substance as a drug that improves skeletal myocyte function by using contractility as an indicator.
[19] A kit for testing cardiotoxicity of a test substance or for screening a drug that improves cardiomyocyte function, comprising the pluripotent stem cell according to any one of [1]-[3], a cardiomyocyte generated by the method according to [8], or a cardiac organoid generated by the method according to [11].
[20] A kit for testing hepatotoxicity of a test substance or for screening a drug that improves hepatocyte function, comprising the pluripotent stem cell according to any one of [1]-[3], a hepatocyte generated by the method according to [9], or a hepatic organoid generated by the method according to [12].
[21] A kit for screening a drug that improves skeletal myocyte function, comprising the pluripotent stem cell according to any one of [1]-[3] or a skeletal myocyte generated by the method according to [13].

### [Advantageous Effects of the Invention]

According to the present invention, polyploid pluripotent stem cells can be obtained by fusing pluripotent stem cells with each other. Polyploid pluripotent stem cells are useful for basic research and drug discovery research using disease models, and also for cell transplantation, because they accurately reflect adult cells and produce highly differentiated and mature cells.

### [BRIEF DESCRIPTION OF DRAWINGS]

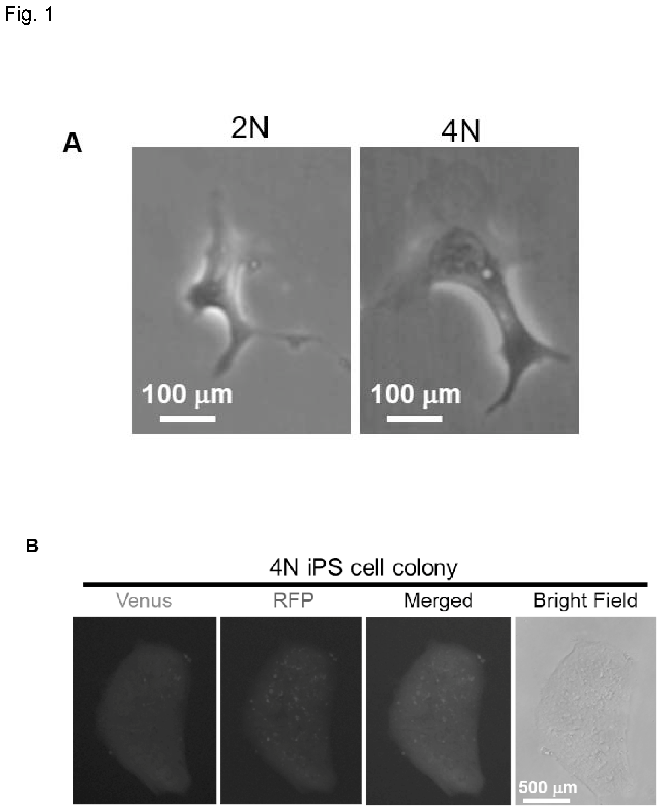

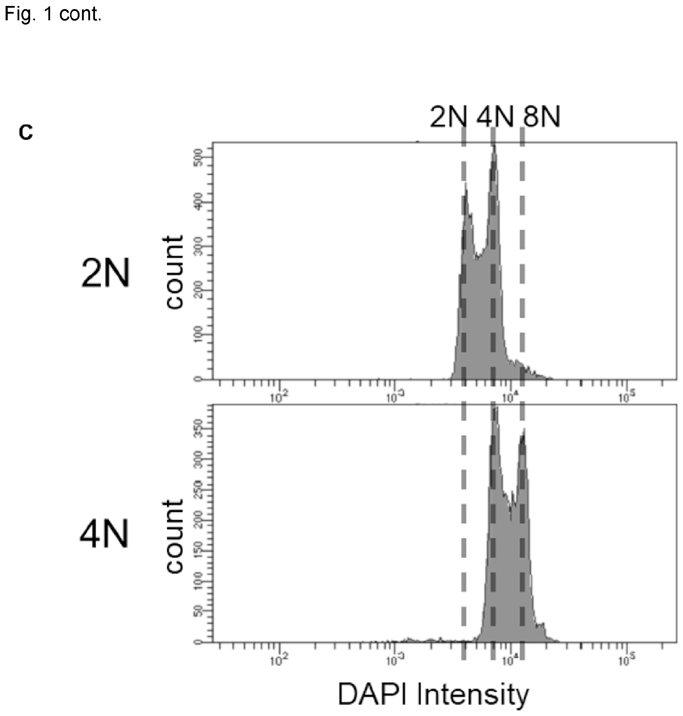

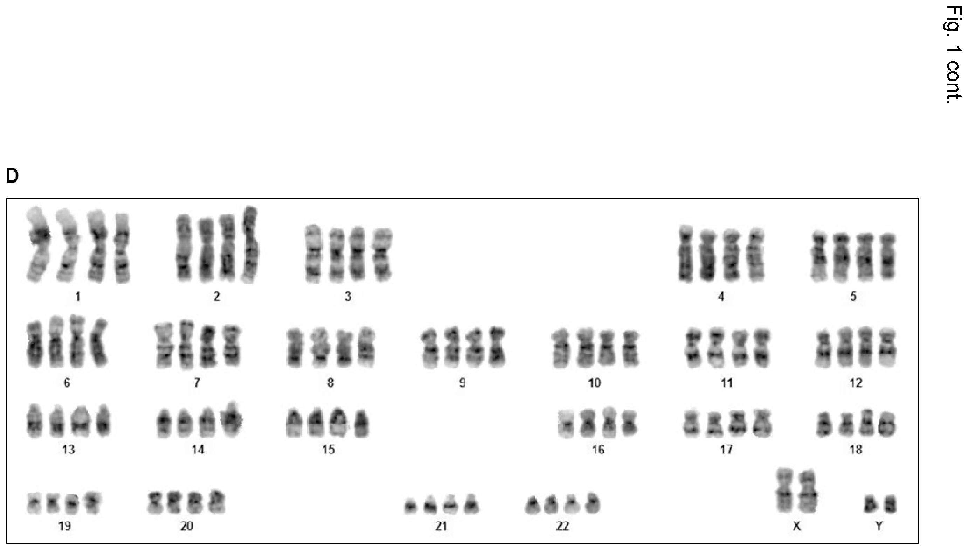

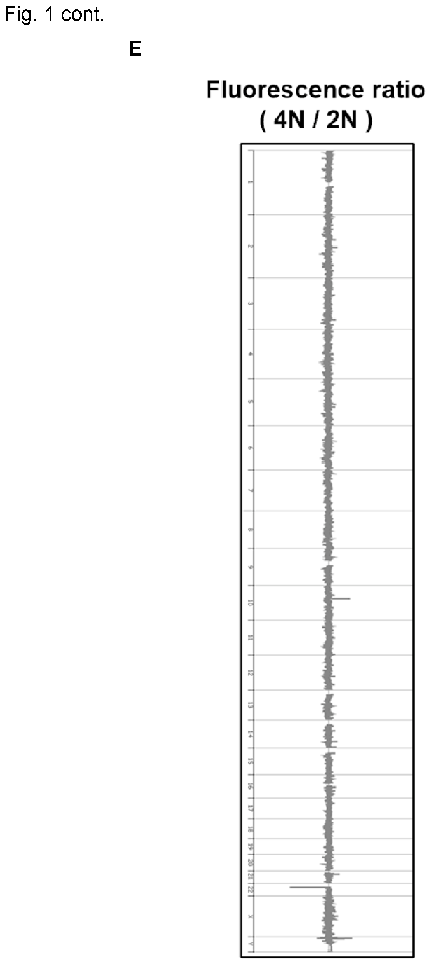

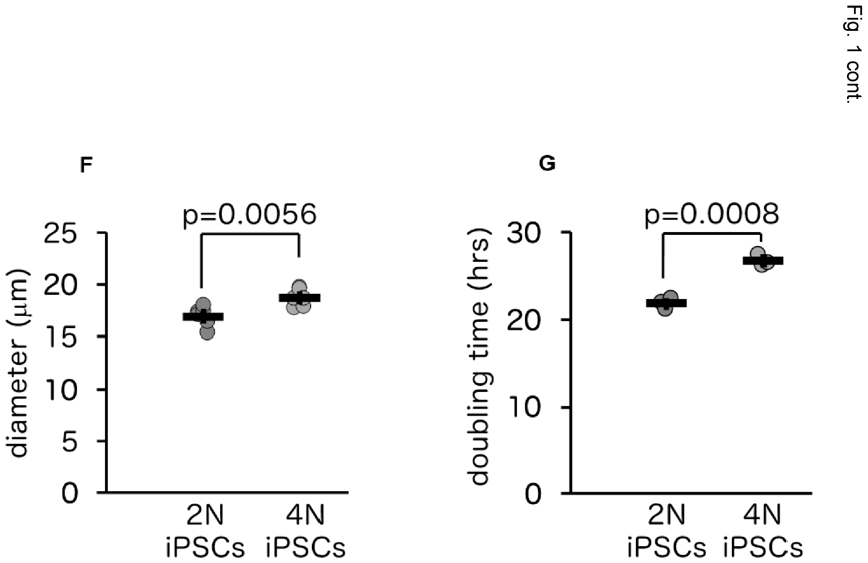

[Figure 1] Diagrams showing the properties of fused tetraploid iPS cells.
   (A) Nuclear staining (blue) of diploid (2N) and tetraploid (4N) iPS cells with Hoescht 33342.
   (B) Venus and mRFP1 expression in tetraploid iPS cell colonies. The tetraploid cells expressed both fluorescent proteins.
   (C) Measurement of the amount of DNA by flow cytometry. The fused tetraploid iPS cells (4N) clearly showed an increased amount of DNA compared to the pre-fused diploid iPS cells (2N).
   (D) Karyotyping of the fused tetraploid iPS cells. Complete chromosome doubling was observed, including sex chromosomes.
   (E) CGH (comparative genomic hybridization) analysis of the tetraploid and diploid iPS cells. There were no microdeletions or insertions, confirming complete genomic DNA doubling.
   (F) Cell diameters of the diploid and tetraploid iPS cells. The tetraploid iPS cells were found to have larger diameters. P-value was calculated by Student's t-test. N = 3.
   (G) Doubling times of the diploid and tetraploid iPS cells. The tetraploid iPS cells were found to show a longer doubling time. P-value was calculated by Student's t-test. N = 3.

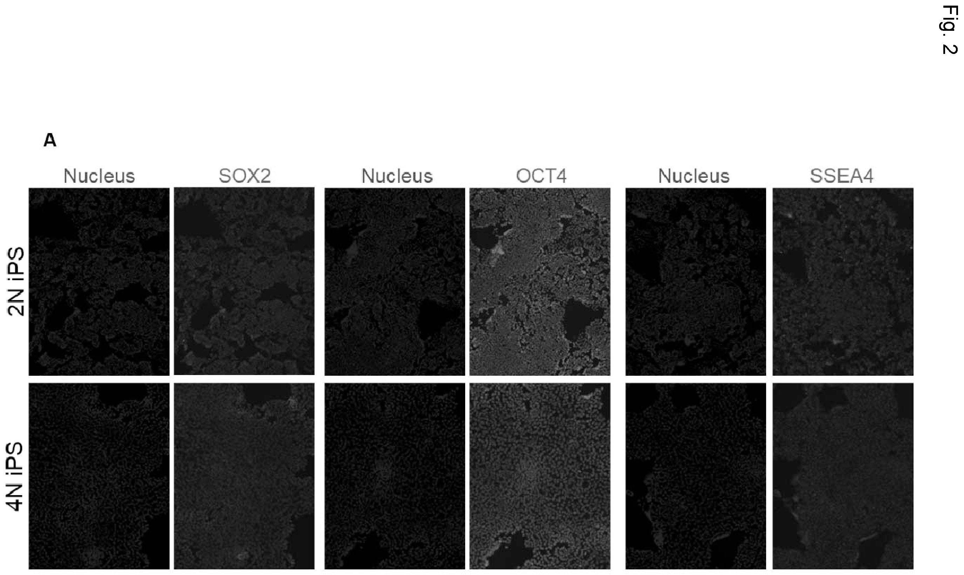

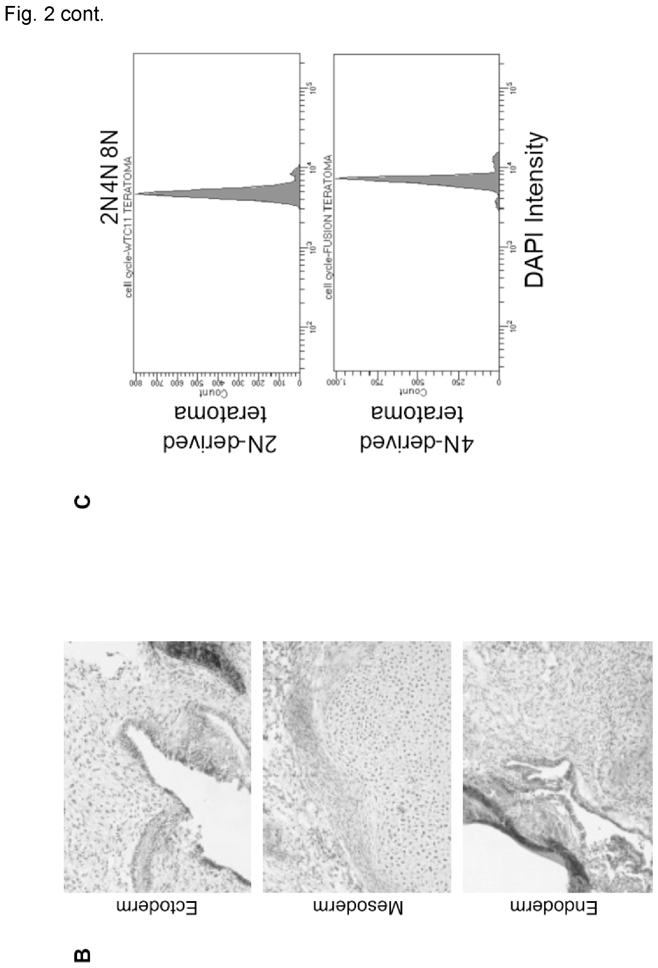

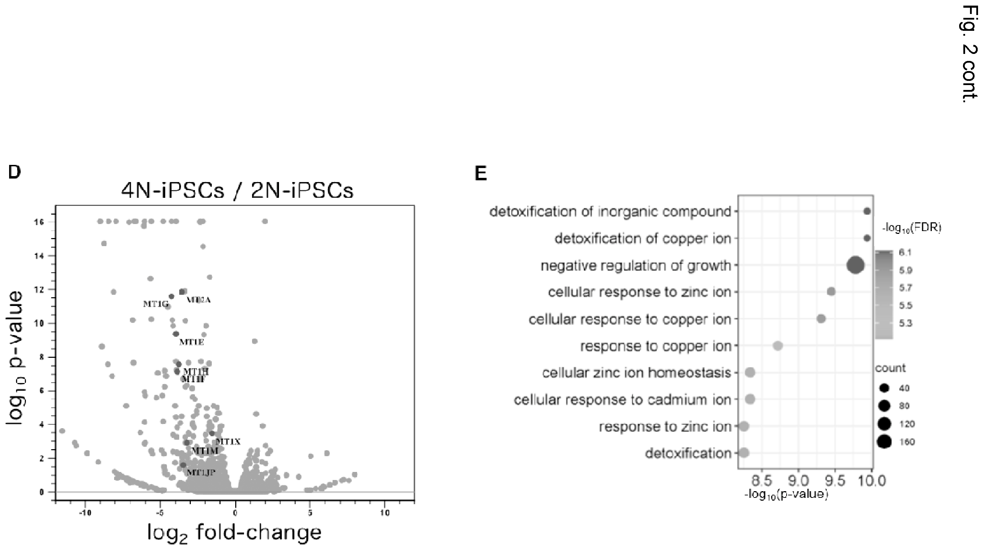

[Figure 2] Diagrams showing the retention of undifferentiated nature of the fused tetraploid iPS cells.
   (A) The diploid (2N) and tetraploid (4N) iPS cells were subjected to immunofluorescent staining (purple) of undifferentiation marker genes and nuclear staining (blue) with Hoescht 33342.
   (B) HE staining of a teratoma formed by transplantation of the tetraploid iPS cells into an immunodeficient mouse. Differentiation into triploblastic cells was observed.
   (C) Ploidy levels of teratoma cells formed from the diploid and tetraploid iPS cells.
   (D) Comparison of gene expressions between the diploid and tetraploid iPS cells by RNA-Seq. Many genes were found to be specifically expressed at low levels only in the tetraploid iPS cells. Metallothionein (MT) genes that respond to oxidative stress and metal attributes are shown in red.
   (E) GO enrichment analysis was used to predict how the changes in the gene expressions shown in Figure (D) would affect the cellular functions. Based on the changes in the MT gene expressions, changes in cellular functions, which respond mainly to oxidative stress and metal toxicity, were predicted.

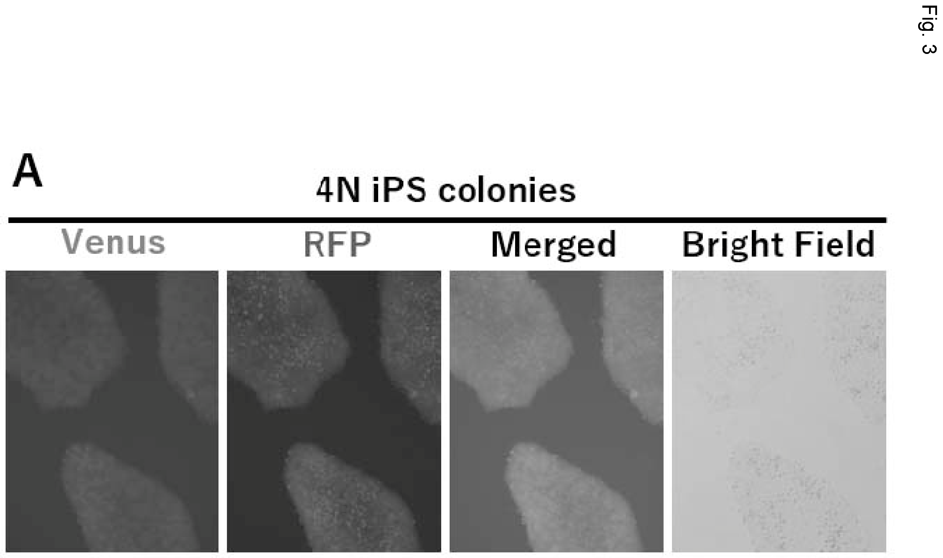

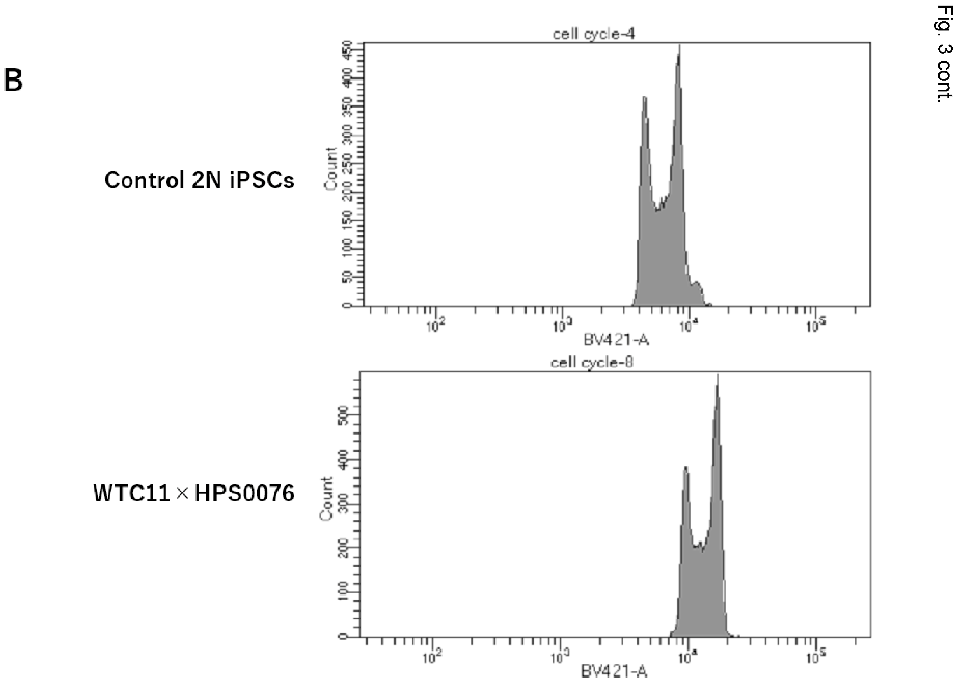

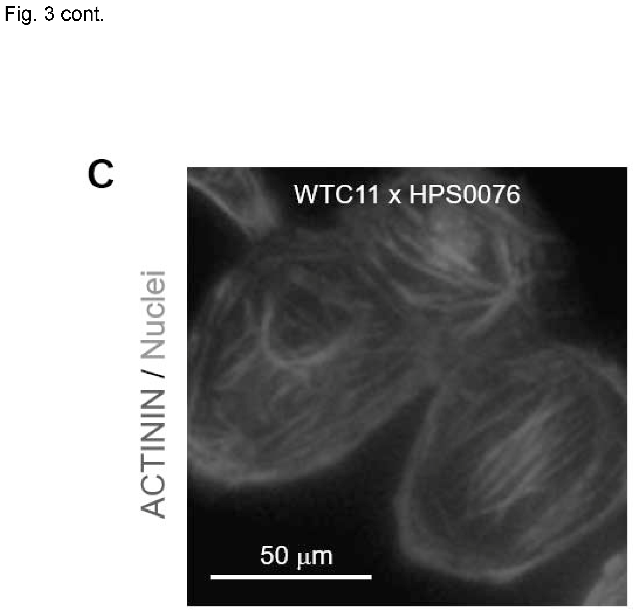

[Figure 3] Diagrams showing the establishment of tetraploid iPS cells through the fusion of diploid iPS cells derived from different individuals.
   (A) Venus and mRFP1 expressed in tetraploid iPS cell colonies established through the fusion of WTC11 and HPS0076 cells. Both fluorescent proteins were expressed.
   (B) Measurement of the amount of DNA by flow cytometry. The fused iPS cells (WTC11×HPS0076) clearly showed an increased amount of DNA compared to the pre-fused diploid WTC11 (Control 2N iPSCs).
   (C) Immunofluorescent staining of actinin in cardiomyocytes derived from the fused iPS cells (WTC11 ×HPS0076). Expression of actinin, i.e., a cardiomyocyte marker, was observed.
[Figure 4] Diagrams showing the results of the generation of octoploid iPS cells through the fusion of tetraploid iPS cells.
   Diagrams show the results of measurement of the amount of DNA by flow cytometry. Compared to the control, the peak (P5) raised when tetraploid iPS cells were fused with each other, indicating the generation of octoploids.
[Figure 5] Diagrams showing the results of the comparison of the amounts of DNA, amounts of mitochondria, and gene expressions between the diploid and tetraploid iPS cell-derived cardiomyocytes.
   (A) Immunofluorescent staining of actinin in cardiomyocytes derived from the diploid and tetraploid iPS cells. Cardiomyocytes derived from the tetraploid iPS cells were larger.
   (B) Measurement of the amount of DNA by flow cytometry.
   (C) Comparison of gene expression profiles between the diploid and tetraploid iPS cell-derived cardiomyocytes.
   (D) Mitochondria (green) staining using MitoTracker and nuclear (blue) staining.
   (E) Quantification of the amount of mitochondria relative to nucleus, using FiJi application. The amount of mitochondria per nucleus (cell) was significantly higher in the tetraploids.
   (F) Ratios of mitochondrial DNA copy number to nuclear DNA copy number in undifferentiated diploid and tetraploid iPS cells, obtained by digital PCR. Fifteen samples each were analyzed, but no significant differences were found.
   (G) Ratios of mitochondrial DNA copy number to nuclear DNA copy number in cardiomyocytes derived from the diploid and tetraploid iPS cells, obtained by digital PCR. Fifteen samples each were analyzed, and a significant increase in the mitochondrial DNA copy number was observed in the tetraploid iPS cell-derived cardiomyocytes.
   (H) GO enrichment analysis was used to predict cellular functions affected by changes in the gene expressions shown in Figure 5C. All of the top ten were related to the cell cycle.

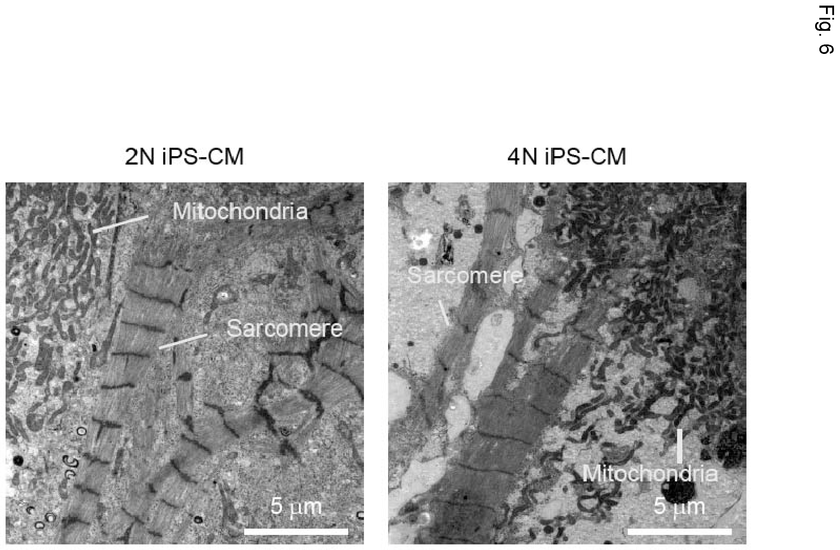

[Figure 6] Transmission electron microscopy analysis of diploid and tetraploid iPS cell-derived cardiomyocytes.

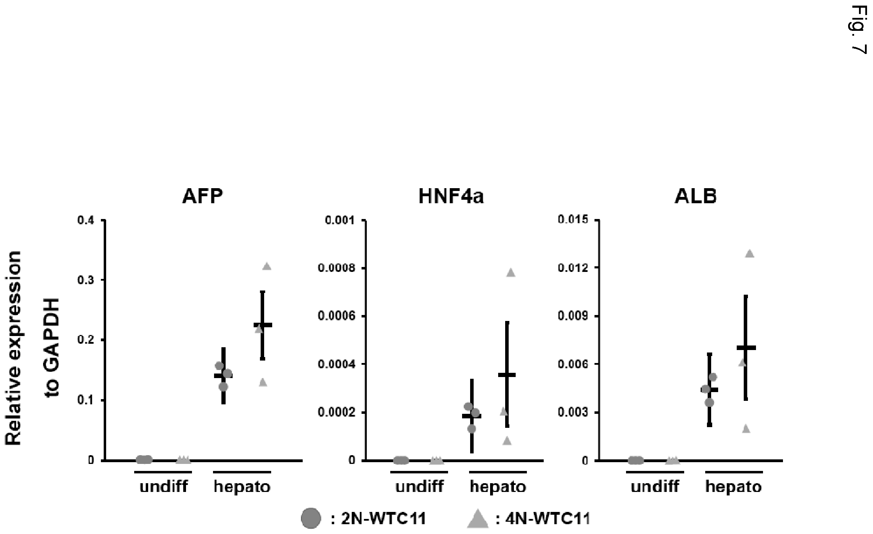

[Figure 7] Diagrams showing the results of quantitative PCR for differentiation marker expressions in diploid and tetraploid iPS cell-derived hepatocytes.
   AFP, HNF4a, and ALB were all highly expressed in the tetraploid iPS cell-derived hepatocytes.

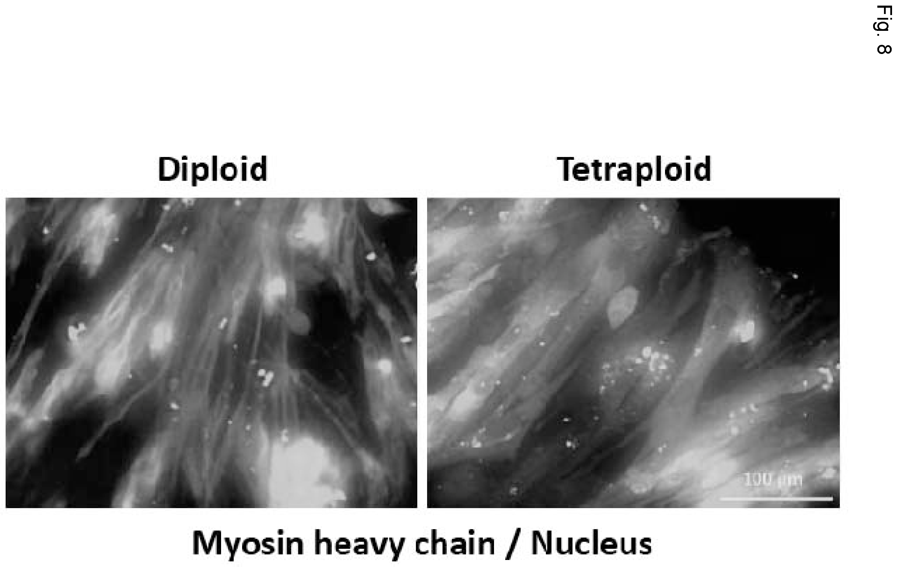

[Figure 8] Diagrams showing myosin heavy chain (green) and nuclear (blue) staining in diploid and tetraploid iPS cell-derived skeletal myocytes.
   Both were positive and fibrous morphology was observed.

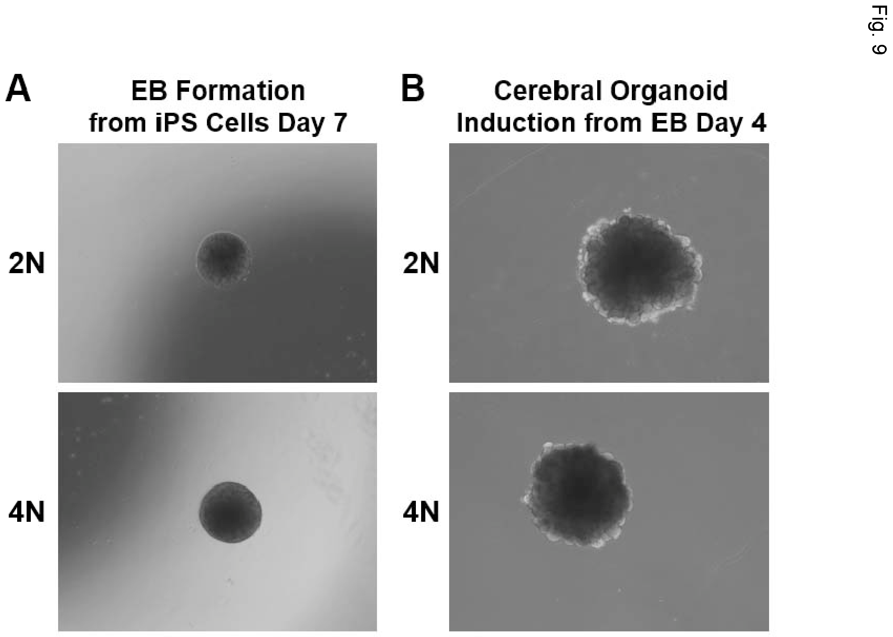

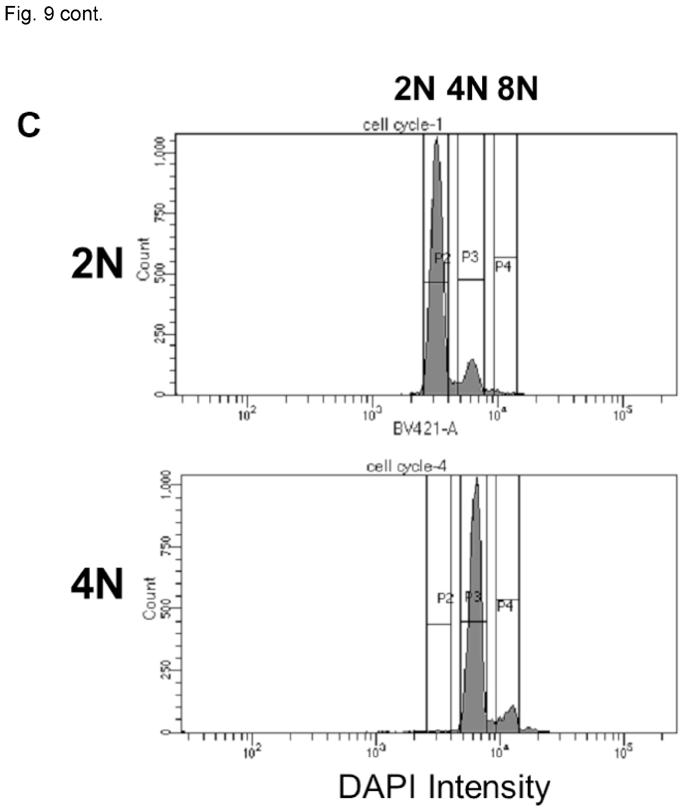

[Figure 9] Diagrams showing EBs and cerebral organoids formed from the diploid and tetraploid iPS cells.
   (A) EBs formed from the diploid and tetraploid iPS cells.
   (B) Cerebral organoids formed from the diploid and tetraploid iPS cells.
   (C) Measurement of the amounts of DNA in the cerebral organoids formed from the diploid and tetraploid iPS cells, by flow cytometry.

### [MODES FOR CARRYING OUT THE INVENTION]

The present invention relates to a polyploid pluripotent stem cell generated through the cell fusion of diploid pluripotent stem cells with each other. The present invention also relates to a method for producing a polyploid pluripotent stem cell, comprising a step of fusing diploid pluripotent stem cells with each other using Sendai virus particles or polyethylene glycol.

### 1. Overview

Instead of inducing differentiation of normal diploid iPS cells into polyploid cell types, the present invention succeeded in fusing pluripotent iPS cells having multipotency with each other in advance to polyploidize them in an undifferentiated state and then inducing them to differentiate into polyploid cell types. Hybridomas, in which B cells are fused with myeloma cells to generate monoclonal antibodies, are the most famous example of cell fusion, and their usefulness is already well known (7). In cases of fusion using iPS cells, there are reports on the establishment of tetraploid cells by fusing iPS cells with another cell type (8, 9). There is also a report on the establishment of triploid ES cells by fusing normal diploid ES cells with haploid ES cells (10). However, triploid cells do not normally exist in the human body. The present invention succeeded in fusing iPS cells retaining the undifferentiated nature with each other to establish tetraploid or octoploid cells, which can be found in normal living organisms, and then inducing them to differentiate into a cell type of interest. The fused cells obtained according to the present invention are useful for basic research and drug discovery research using disease models, and also for cell transplantation, because they accurately reflect adult cells and produce highly differentiated and mature cells, making them useful for regenerative medicine.

### 2. Pluripotent stem cells

The type of pluripotent stem cells to be fused is not particularly limited, and any pluripotent stem cells from humans or non-human mammals, such as ES cells (Embryonic Stem cells), iPS cells (induced Pluripotent Stem cells), etc. can be used. These pluripotent stem cells can also be transfected with a gene that expresses a fluorescent protein and/or a drug-resistant gene to facilitate confirmation of the cell fusion and confirmation of the subsequent cell behavior, differentiation, etc. Examples of the fluorescent protein include red fluorescent proteins (mRFP1, Ds-Red, mCherry), green fluorescent proteins (Venus, EGFP, GFP2), and blue fluorescent proteins (Sirius, ECFP, EBFP2).

Meanwhile, examples of the drug-resistant gene include puromycin-resistant genes, blasticidin-resistant genes, neomycin-resistant genes, and hygromycin-resistant genes.

Note that the fluorescent proteins and the drug-resistant genes are not limited to those listed above, and they may be selected and used as appropriate.

Gene transfer can be performed by a known technique, and an expression vector capable of expressing the protein can be introduced. The expression vector may be linked with a suitable promoter (CMV promoter, EF1-α promoter, etc.), other RNA element that allows initiation of cap-independent translation (e.g., IRES (internal ribosome entry site), 2A peptide), and the like.

For the introduction of the above fluorescent protein or drug-resistant gene into pluripotent stem cells, any method available to those skilled in the art can be employed, such as a method using a viral vector, an electroporation method, and a lipofection method.

### 3. Cell fusion

The present invention is a (N₁ + N₂)-ploid pluripotent stem cell comprising a fused cell of a N₁-ploid (wherein N₁ represents an integer from 2 to 16) pluripotent stem cell and a N₂-ploid (wherein N₂ represents an integer from 2 to 16) pluripotent stem cell. The (N₁ + N₂)-ploid pluripotent stem cell of the present invention is a (N₁ + N₂)-ploid (polyploid) pluripotent stem cell which is obtained by culturing and thus fusing a N₁-ploid (wherein N₁ represents an integer from 2 to 16) pluripotent stem cell and a N₂-ploid (wherein N₂ represents an integer from 2 to 16) pluripotent stem cell in the presence of a virus. The present invention also relates to a method for producing a (N₁ + N₂)-ploid pluripotent stem cell, comprising a step of culturing a N₁-ploid (wherein N₁ represents an integer from 2 to 16) pluripotent stem cell and a N₂-ploid (wherein N₂ represents an integer from 2 to 16) pluripotent stem cell in the presence of a virus.

If both N₁ and N₂ are diploids, the cell fusion results in a tetraploid; if N₁ is 2 and N₂ is 3, the cell fusion results in a pentaploid. Haploid human cells, for example, human haploid ES cells, are known (e.g., Ido Sagi et al., Nature volume 532, pages 107-111 (2016)). Therefore, a cell, in which N₁ or N₂ is a triploid, can be generated by fusing the above haploid ES cell with a diploid cell.

Moreover, for example, a tetraploid (N₁ = 4) can be produced by fusing diploid parental strains with each other, which is then fused again with a diploid parental strain (N₂ = 2) to give a hexaploid.

In this manner, pluripotent stem cells of any polyploidy (up to a dotriacontaploid) can be generated according to the present invention, but a tetraploid obtained by fusing diploids with each other or an octoploid obtained by fusing tetraploids with each other, is preferred.

Cell fusion can be performed, for example, by fusing about 2 × 10⁵ pluripotent stem cells with each other using HVJ-E (Ishihara Sangyo Kaisha, Ltd.), a Sendai virus particle fusion technique. The number of cells to be fused with each other may be the same or the number of cells may be altered suitably by changing the mixing ratio. The number of cells can also be adjusted according to the scale of culture following fusion.

The culture medium used may be a medium commonly used for iPS cell culture, such as mTeSR Plus (STEMCELL TECHNOLOGY) and StemFit (Ajinomoto). Note that the fusion itself is performed in the presence of the cell fusion buffer provided with the HVJ-E kit.

When culturing the fused cells, it can be performed in the same manner as with normal diploid iPS cells in a basic medium.

In order to obtain a fused cell of interest, it is necessary to select a cell that has become drug-resistant through the fusion of pluripotent stem cells. For this purpose, one pluripotent stem cell (referred to as "pluripotent stem cell 1") is transfected with a gene (referred to as "drug-resistant gene 1") which is resistant to one drug (referred to as "drug 1") while the other pluripotent stem cell (referred to as "pluripotent stem cell 2") is transfected with a gene (referred to as "drug-resistant gene 2") which is resistant to another drug (referred to as "drug 2"). Fused cells can be sorted out by adding drug 1 and drug 2 to a medium and obtaining cells resistant to both drugs.

If pluripotent stem cells 1 are labeled with a red fluorescent protein and pluripotent stem cells 2 are labeled with a green fluorescent protein, the fused cells will express both fluorescent proteins and thus can be separated using a cell sorter or the like. In this way, the fused cells of the present invention can be isolated.

Among the fused cells obtained by the present invention, polyploid pluripotent stem cells have the following characteristics.
- They are larger in size than normal diploid cells and are mononuclear.
- The amount of DNA in the pluripotent stem cells is doubled.
- They retain completely doubled chromosomes with no genetic abnormalities.
- The proliferation rate is slightly slower than that of diploid cells.
- Expressions of the undifferentiation markers are retained.
- They have multipotency, and retain the ploidy level (e.g., tetraploidy) even after differentiation.
- The ploidy level can be increased, for example, to an octoploid or a hexadecaploid.

### 4. Differentiation of fused cells

According to the present invention, fused cells obtained as described above can be induced to differentiate into various tissues *in vivo* or *in vitro.* As described above, since the fused tetraploid cells retain their ploidy level, undifferentiated nature, and multipotency, it is possible to generate a model tissue that closely resemble the real tissue by transplanting the fused cells into an animal or by culturing them in the presence of a differentiation factor (differentiation in a two-dimensional medium).

For example, the fused cells of the present invention can be cultured in the presence of various drugs or factors so that they differentiate into cardiomyocytes or a myocardial tissue, hepatocytes or a liver tissue, a cerebral tissue, or skeletal myocytes or a skeletal muscle tissue. In one aspect of the present invention, a myocardial tissue model can be generated *in vitro* by treating the fused cells of the present invention with a GSK-3 inhibitor, which is a potent pharmacological activator of Wnt/β-catenin signaling pathway. In addition, a liver tissue model can be generated *in vitro* by inducing the fused cells of the present invention to differentiate into endodermal cells by treating with Activin A and subsequently treating with FGF, BMP, and HGF factors.

Furthermore, according to the present invention, skeletal myocytes can be generated by subjecting the fused cells of the present invention to forced expression of MYOD1 gene or treatment with a TGF-β receptor inhibitor.

Alternatively, the fused cells can be transplanted into a non-human mammal to form any kind of tissue at the transplant site, and this animal can be used as a model animal. Examples of the animal include, but are not limited to, a mouse, a rat, a rabbit, a guinea pig, and a monkey. In general, it is desirable to select an animal species in which the fused cells are viable. When transplanting the above fused cells into an animal, the fused cells can be suspended, for example, in phosphate-buffered saline (PBS), or a semi-solid medium such as collagen or Matrigel can be used to improve the transplant and patient survival rates. The formation of various tissues can be confirmed or evaluated through macroscopic observation of cell morphology, expression of marker genes in each tissue, expression or secretion of a protein specific to that tissue, and the like.

### 5. Generation of organoid

An organoid is a small organ generated *in vitro* from stem cells. An organoid is formed as a three-dimensional tissue-like structure through self-organization, utilizing the self-renewal and differentiation potential of stem cells.

### (1) Cardiac organoid

A cardiac organoid can be produced, for example, by the following steps.

First, a polyploid pluripotent stem cells are cultured on a low attachment plate to induce them into an embryoid body. The resulting embryoid body is then treated with a GSK-3 inhibitor (e.g., CHIR99021), an osteogenic factor (e.g., BMP-4), and Activin A to induce it into a mesoderm, which is further treated with Wnt-C59 to induce it into a cardiac mesoderm. The induced cardiac mesoderm is then treated with a GSK-3 inhibitor.

### (2) Hepatic organoid

A hepatic organoid can be produced, for example, by the following steps.

The polyploid pluripotent stem cells of the present invention are cultured together (co-cultured) with iPS cell-derived endodermal cells which are induced in two-dimensions, vascular endothelial cells, and mesenchymal stem cells to allow self-aggregation to generate a three-dimensional hepatic organoid.

### (3) Cerebral organoid

A cerebral organoid can be produced from the polyploid pluripotent stem cells of the present invention by using, for example, STEMdiff^{™} Cerebral Organoid Kit (STEMCELL TECHNOLOGIES). Specifically, an embryoid body (EB) is generated by a floating culture treatment with a basic fibroblast growth factor (bFGF) and a rho-associated protein kinase (ROCK) inhibitor (Ri). Next, the EB is induced into a neuroepithelium using a medium containing N2 supplement, e.g., a DMEM-F12 medium containing N2 supplement, GlutaMAX, MEM-NEAA, and heparin. The resultant is further induced into a cerebrum by a treatment with a mixed medium of a neurobasal medium and a medium containing N2 supplement and B27 supplement, e.g., a DMEM-F12 medium containing N2 supplement, insulin, Glutamax, MEM-NEAA, 2-mercaptoethanol, and B27 supplement. The three-dimensional tissue is matured by subsequent shaking culture.

### (4) Skeletal muscle organoid

A skeletal muscle organoid can be produced, for example, by the following steps.

After inducing the polyploid pluripotent stem cells of the present invention into iPS cell-derived skeletal myocytes in two-dimensions, they are allowed to coagulate, together with fibroblasts, around a pillar device consisting of two plastic and flexible materials using fibrinogen and thrombin to generate a three-dimensional skeletal muscle organoid.

### 6. Screening methods and toxicity test methods

According to the present invention, toxicity of a test substance can be tested using the above pluripotent stem cells, cardiomyocytes, cardiac organoid, hepatocytes, hepatic organoid, cerebral organoid, skeletal myocytes, skeletal muscle organoid, etc. Alternatively, a drug that improves cardiomyocyte function, a drug that improves hepatocyte function, a drug that improves brain function, or a drug that improves skeletal myocyte function can be screened.

A method for testing cardiotoxicity comprises the steps of: contacting a test substance with the pluripotent stem cells, cardiomyocytes, or cardiac organoid described above; and evaluating the test substance as a cardiotoxic substance by using cell death, cell membrane potential, calcium transient, or reduced muscle contractility as an indicator.

A method for screening a drug that improves cardiomyocyte function, comprises the steps of: contacting a test substance with the pluripotent stem cells, cardiomyocytes, or cardiac organoid described above; and selecting the test substance as a drug that improves cardiomyocyte function by using muscle contractility, calcium transient, or beating rhythm as an indicator.

A method for testing hepatotoxicity comprises the steps of: contacting a test substance with the pluripotent stem cells, hepatocytes, or hepatic organoid described above; and evaluating the test substance as a hepatotoxic substance by using cell death or mitochondrial toxicity as an indicator.

A method for screening a drug that improves hepatocyte function comprises the steps of: contacting a test substance with the pluripotent stem cells, hepatocytes, or hepatic organoid described above; and selecting the test substance as a drug that improves hepatocyte function by using drug-metabolizing ability as an indicator.

The term "contact" means placing a candidate substance (test substance) with pluripotent stem cells, cardiomyocytes, hepatocytes, skeletal myocytes, a cardiac organoid, a hepatic organoid, a cerebral organoid, or a skeletal muscle organoid in the same environment, reaction system or culture system. For example, the term includes adding a candidate substance to a cell culture vessel, mixing cells or an organoid with a candidate substance, or culturing cells or an organoid in the presence of a candidate substance.

Examples of the candidate substance include peptides, proteins (including antibodies), non-peptide compounds, synthetic compounds (high or low molecular weight compounds), fermentation products, cell extracts, cell culture supernatants, plant extracts, tissue extracts from mammals (e.g., mouse, rat, pig, cow, sheep, monkey, human, etc.), and plasmas, where these compounds may be novel or known compounds. These candidate substances may form salts. The salts of the candidate substances are salts of physiologically acceptable acids (e.g., inorganic acids, organic acids, etc.) or bases (e.g., metallic acids, etc.).

If contacting a candidate substance with the pluripotent stem cells, the cardiomyocytes, or the cardiac organoid results in cell death, cell membrane potential, calcium transient, or reduced muscle contractility, the candidate substance used can be judged or evaluated as a cardiotoxic substance.

Cell death can serve as an indicator of cardiotoxicity and can be detected by a method in which dead cells are stained with trypan blue, propidium iodide (PI), etc., by a method in which cells are labeled with a radioisotope (e.g., 51Cr, etc.) in advance and the radioisotope that is released upon cell death is measured, by a method in which a modified dUTP attached to DNA, which is highly fragmented due to cell death, is detected (TUNEL assay), by ELISA using fragmented DNA and an antibody against a histone protein bound to the DNA, or by a method in which lactate dehydrogenase released from damaged cells into a culture medium is measured.

Cell membrane potential can serve as an indicator of cardiotoxicity, when action potentials are measured using a patch clamp technique, a multi-electrode system, or a membrane potential-sensitive dye, or the like, and if the measurement results show detection of a greater incidence of prolonged FPD (field potential duration), appearance of TdP (torsade de pointes)-like waveform, or appearance of an abnormal waveform such as EAD (early afterdepolarization) or triggered activity, compared to the control group.

Calcium transient can serve as an indicator of cardiotoxicity, when the calcium ions entering the cells are measured using a Ca²⁺-sensitive fluorescent indicator, etc., and if change in the calcium transient waveform or waveform peak is higher or lower than the control group by 20% or more.

The method for screening a drug that improves cardiomyocyte function involves bringing a candidate substance into contact with the pluripotent stem cells, the cardiomyocytes, or the cardiac organoid, and if the result confirms muscle contractility or beating rhythm, the candidate substance used can be chosen as a drug that improves cardiomyocyte function.

Muscle contractility is determined by generating three-dimensional cardiomyocytes around a pillar device and measuring the distance the pillar device moves due to cardiomyocyte contractility. If the distance the pillar device moves is longer than that of the control group, it serves as an indicator of a drug that improves cardiomyocyte function. Alternatively, cardiomyocytes are cultured on a plastic extracellular matrix, and contractility is measured using the degree of deformation as an indicator. A greater degree of deformation serves as an indicator of cardiotoxicity.

Beating rhythm can serve as an indicator of a drug that improves cardiomyocyte function, when the number of beats of a two- or three-dimensional cardiomyocyte tissue is counted, and if the beating rhythm is constant compared to the control group.

If contacting a candidate substance with the pluripotent stem cells, the hepatocytes, or the hepatic organoid results in cell death or mitochondrial toxicity, the candidate substance used can be judged or evaluated as a hepatotoxic substance.

Cell death can serve as an indicator of hepatotoxicity and can be detected by a method in which dead cells are stained with trypan blue, propidium iodide (PI), etc., by a method in which cells are labeled with a radioisotope (e.g., 51Cr, etc.) in advance and the radioisotope that is released upon cell death is measured, by a method in which a modified dUTP attached to DNA, which is highly fragmented due to cell death, is detected (TUNEL assay), by ELISA using fragmented DNA and an antibody against a histone protein bound to the DNA, or by a method in which lactate dehydrogenase released from damaged cells into a culture medium is measured.

Mitochondrial toxicity can serve as an indicator of hepatotoxicity. This is true when the mitochondrial membrane potential, ATP content, glutathione concentration, etc. are measured, and the measured result is lower than those of the control group by 20% or more.

The method for screening a drug that improves hepatocyte function involves bringing a candidate substance into contact with the pluripotent stem cells, the hepatocytes, or the hepatic organoid, and if the result confirms drug-metabolizing ability, the candidate substance used can be chosen as a drug that improves hepatocyte function.

Drug-metabolizing ability can serve as an indicator of a drug that improves hepatocyte function. This is true when cytochrome P450 (CYP) enzyme activity, CYP gene expression level, or glucuronosyl transferase (UGT) activity is measured, and an increase in the enzyme activity or gene expression is confirmed.

Examples of the drug-metabolizing ability as an indicator include CYP, UGT, esterase, flavin-containing monooxygenase, N-acetyltransferase, and monoamine oxidase.

The method for screening a drug that improves skeletal myocyte function involves bringing a candidate substance into contact with the pluripotent stem cells, the skeletal myocytes, or the skeletal muscle organoid, and if the result confirms improved contractility, the candidate substance used can be chosen as a drug that improves skeletal myocyte function.

Contractility can be measured by generating a skeletal muscle organoid, applying electric pulses thereto, and measuring the distance the pillar device moves. If the distance is longer than that of the control group, it serves as an indicator of a drug that improves skeletal myocyte function.

The differentiation/maturity level can also be measured based on the sarcomere structure and gene expression. If clearly observed under an electron microscope, the light and dark bands and the z-membrane can be an indicator of improved skeletal myocyte function. As to gene expression, an increased gene expression of, for example, MHC, SCN4A, MYH, etc., is an indicator of improved skeletal myocyte function.

### 7. Screening kit

The pluripotent stem cells, cardiomyocytes, or cardiac organoid of the present invention can be provided in the form of a kit for evaluating cardiotoxicity or a kit for screening a drug that improves cardiomyocyte function.

The pluripotent stem cells, hepatocytes, or hepatic organoid of the present invention can also be provided in the form of a kit for evaluating hepatotoxicity or a kit for screening a drug that improves hepatocyte function.

The pluripotent stem cells, skeletal myocytes, or skeletal muscle organoid of the present invention can also be provided in the form of a kit for screening a drug that improves skeletal myocyte function.

The kit of the present invention includes the cells or organoid described above, and can also contain a labeling substance, a reagent for detecting cell death (e.g., LDH, etc.), and the like. A labeling substance refers to an enzyme, a radioisotope, a fluorescent compound, a chemiluminescent compound, or the like. In addition to the above components, the kit of the present invention can also contain other reagents for carrying out the method of the invention. For example, if the labeling is enzyme labeling, it may contain an enzyme substrate (e.g., chromogenic substrate), an enzyme substrate solution, an enzyme reaction stopping solution, or the like. The kit of the present invention may further include a diluent for test compounds, various buffers, sterile water, various cell culture vessels, various reaction vessels (Eppendorf tube, etc.), a detergent, an experimental operations manual (instructions), and the like.

### EXAMPLES

Hereinafter, the present invention will be described in further detail by way of examples. The scope of the invention, however, should not be limited to these examples.

### [Example 1]

### Methods

### Culture of human iPS cells

As normal diploid human iPS cells, WTC11, which were established episomally from a healthy Japanese male with informed consent (11), and HPS0076, which were iPS cells derived from a white female obtained from the Riken cell bank, were used. They were cultured on a plastic dish coated with GFR Matrigel Phenol Red-Free using mTeSR Plus + 1% penicillin-streptomycin (mTeSR Plus+Pen/Strep), which is a common medium for iPS cells. While the cell density was low, ROCK inhibitor Y-27632 (ROCK inhibitor, Ri) was added to the medium at 10 µM. The same conditions were used to culture tetraploid and octoploid iPS cells after fusion.

### Lentiviral transduction of fluorescent protein and drug-resistant genes into iPS cells

Lentiviruses used were generated by introducing a puromycin-resistant gene (PuroR), a blasticidin-resistant gene (BsdR), a neomycin-resistant gene (NeoR), a hygromycin-resistant gene (HygR), and EF1α promoter into lentiviral vectors CSII-CMV-MCS-IRES2-Venus and CSII-CMV-MCS-IRES2-mRFP1 (RIKEN) (CSII-EF1α-VENUS-IRES2-PuroR, CSII-EF1α-mRFP1-IRES2-BsdR, CSII-EF1α-NeoR, CSII-EF1α-HygR).

Two 10 cm dishes were each seeded with 4.0 x 10⁶ HEK293 cells. After 17 hours, the medium was replaced with new DMEM + 10% FBS. Six hours later, 1,800 µl of Opti-MEM (Thermo Fisher Scientific) was mixed with 5.4 µg of pMDLg/p-RPE (RIKEN) as a lentiviral packaging plasmid, 4.5 µg of pCMV-VSV-G-RSV-Rev (RIKEN) as a lentiviral envelope plasmid, 8.1 µg of the prepared lentiviral vector, and 54 µl of Polyethylenimine (PEI) Max (Polysciences). The resultant was kept at room temperature for 25 minutes, and dropped into the medium. After 16 hours, the medium was replaced with DMEM + 10% FBS + 10 mM HEPES Buffer (Thermo Fisher Scientific). After 55 hours, the medium was collected and centrifuged at 4°C and 600 g for 20 minutes. The supernatant was filtered using a 0.45 µm filter and a 20 ml syringe, collected in a new tube, and centrifuged at 4°C and 5,800 g overnight. The supernatant was removed and 100 µl of mTeSR Plus was added to the precipitate and mixed. After shaking at 4°C for 8 hours, the mixture was resuspended. The suspension was centrifuged at 4°C and 600 g for 2 minute, dispensed into tubes, and stored at -80°C.

1.25 × 10⁵ iPS cells were seeded on a 12-well plate coated with Matrigel. After 24 hours, the medium was replaced with mTeSR Plus+Pen/Strep w/o Ri containing 25 µl of virus solution. Once the cells reached confluency, the well was washed three times with PBS. The expression of the fluorescent protein was confirmed under a fluorescence microscope, the medium was replaced with mTeSR Plus + Pen/Strep+Ri containing puromycin at a concentration of 0.5 µg/mL or blastcidin at a concentration of 10 µg/mL, and the cells of interest were selected.

WTC11 cells transduced with mRFP1-IRES2-BsdR or VENUS-IRES2-PuroR using the lentivirus are referred to as "WTC11+mRFP1-IRES2-BsdR" and "WTC11+VENUS-IRES2-PuroR", respectively.

Additionally, HPS0076 cells transduced with mRFP1-IRES2-BsdR or VENUS-IRES2-PuroR using lentivirus are referred to as "HPS0076+mRFPI-IRES2-BsdR" and "HPS0076+VENUS-IRES2-PuroR", respectively.

In addition, tetraploid WTC11 cells transfected with NeoR or HygR are referred to as "4N-WTC11+NeoR" and "4N-WTC11+HygR", respectively.

### Fusion of iPS cells and drug selection

To generate tetraploid iPS cells, cells of either WTC11+mRFP1-IRES2-BsdR or HPS0076+mRFP1-IRES2-BsdR, as well as cells of either WTC11+VENUS-IRES2-PuroR or HPS0076+VENUS-IRES2-PuroR, were detached from the culture dishes, and 2.0 × 10⁵ cells of each were suspended in PBS, combined in one tube, and mixed thoroughly. The resultant was centrifuged at 4°C and 120 g for 3 minutes, the supernatant was removed, and 50 µl of Cell fusion buffer (Ishihara Sangyo Kaisha, Ltd.) was added to the pellets to thoroughly suspend them. 1 µl of ice-cold HVJ-E (Ishihara Sangyo Kaisha, Ltd.) was added as Sendai virus for cell fusion, suspended thoroughly, and kept in ice for 5 minutes. After centrifugation at 4°C and 780 g for 5 minutes, the resultant was kept warm at 37°C for 15 minutes. After adding 10 ml of mTeSR Plus+Pen/Strep+Ri to suspend the cells, the cell suspension was seeded into a 96-well plate at 100 µl/well and cultured continuously. Once the fused cells were confirmed to have grown sufficiently with a fluorescence microscope, the two types of drugs were added to sort out the fused cells. In the same manner, 4N-WTC11-NeoR and 4N-WTC11+HygR were subjected to cell fusion to generate octoploid iPS cells.

To generate tetraploid iPS cells using polyethylene glycol, cells of either WTC11/HPS0076+mRFP1-IRES2-BsdR, as well as cells of either WTC11/HPS0076+VENUS-IRES2-PuroR, were detached from the culture dishes, and 2.0 × 10⁵ cells of each were suspended in PBS, combined in one tube, and mixed thoroughly. The resultant was centrifuged at 4°C and 120 g for 3 minutes, the supernatant was removed, PEG1500 (Roche) was added to the pellets, and the resultant was suspended and incubated at 37°C for 5 minutes. After the resultant was centrifuged at 780 g for 5 minutes, the supernatant was removed. After adding 10 ml of mTeSR Plus+Pen/Strep+Ri to suspend the cells, the cell suspension was seeded into a 96-well plate at 100 µl/well and cultured continuously. Once the fused cells had grown sufficiently under the fluorescence microscope, the two types of drugs were added to sort out the fused cells.

### Immunofluorescent staining

Expressions of undifferentiation marker genes were confirmed by immunofluorescent staining. The tetraploid iPS cells were fixed with 4% paraformaldehyde at room temperature for 20 minutes. After washing the cells with PBS, a blocking solution (3% Goat serum (Jackson ImmunoResearch), 2% Triton-X (Wako) in PBS) was added, and the mixture was incubated at room temperature for 30 minutes. The blocking solution was removed and the resultant was incubated with primary antibodies (SOX2: Abcam#ab97959 (1/500 dilution), OCT4: Abcam#ab19857 (1/500 dilution), SSEA4: Abcam#ab16287 (1/500 dilution), and TRA-1-81: Abcam#ab16289 (1/500 dilution), diluted with a blocking solution) at 4°C overnight in the dark. After washing the cells with PBS, the resultant was incubated with secondary antibodies (Alexa Fluor 647: #A-21235, #A-21244 (1/2000 dilution)) at room temperature for 1 hour in the dark. After washing the cells with PBS, a nuclear staining solution (Hoechst 33342 (Cell Signaling Technology), 1000-fold dilution) was added, and the cells were incubated at room temperature for 10 minutes in the dark. The cells were washed with PBS and then observed under a fluorescence microscope.

### Karyotyping and comparative genome hybridization (CGH) analysis

### Karyotyping was performed by G-band analysis.

For CGH analysis, genomic DNA extracted from iPS cells using DNeasy Blood & Tissue Kit (QIAGEN, 69504) was used. The analysis was performed using SurePrint G3 Human CGH Microarray 4 × 180K (Agilent).

### Measurement of amount of DNA in cells by flow cytometry

iPS cells (5.0 × 10⁵ cells) were suspended in PBS and placed in a 1.5 ml tube. The resultant was centrifuged at 300 g for 5 minutes, and the supernatant was removed. The cells were washed with 1 ml of PBS and centrifuged again to remove the supernatant. 4% Paraformaldehyde was added for fixation at room temperature for 20 minutes. The cells were washed with PBS, and centrifuged to remove the supernatant. After adding 500 µl of PBS to suspend the cells, 5 µl of a cell cycle assay solution (Dojindo Laboratories) was added and the resultant was incubated at 37°C for 15 minutes. The entire amount was transferred to a 35 µm cell strainer to measure fluorescence with a flow cytometer.

### Teratoma formation and histological analysis thereof

Cells on a 6-well plate were prepared for transplantation as follows. The medium was removed, 500 µl of 0.5 mM EDTA (Nacalai Tesque, Inc.) was added, the temperature was kept at 37°C for 10 minutes, and 1.5 ml of mTeSR Plus was added. The cells were gently detached from the plate using Cell Lifter. 1.0 × 10⁶ cells were transferred into a 1.5 ml tube, and centrifuged at room temperature and 200 g for 3 minutes to remove the supernatant. After the pellets were loosened by tapping, 500 µl of 50% Matrigel-50% mTeSR Plus+Ri was added to gently suspend the pellets. Cell suspension was sucked with a 20-gauge needle and a 1 ml syringe, and transplanted into the testes of 8-week-old immunocompromised mice.

Eight weeks after transplantation, autopsies were performed to collect the teratomas. After washing the tissues with PBS, they were fixed with 4% paraformaldehyde at 4°C overnight. The tissues were rinsed under running water for 10 minutes, which was then replaced with 20% sucrose in PBS at 4°C overnight. The solution was changed to 30% sucrose in PBS to allow replacement at 4°C overnight. Tissues were frozen and a cryostat was used to obtain frozen sections with a thickness of 6 µl. Hematoxylin and eosin staining was performed to observe the triploblastic tissue.

### Measurement of cell growth rate

1.0 × 10⁵ cells were suspended in 2 ml of mTeSR Plus+Pen/Strep+Ri and seeded in a 6-well plate. The medium was replaced with mTeSR Plus+Pen/Strep every 2 days. Once the cells reached 90-100% confluency, the number of cells were counted. The seeding, culturing, and counting mentioned above were continued for 60 days to calculate the growth rate.

### Measurement of cell diameter

The diploid WTC11 or tetraploid WTC11 iPS cells were detached from the culture dish and suspended in PBS, and dead cells were stained with 0.4% Trypan blue. Subsequently, the diameter of viable cells was measured using Countess^{™} II Automated Cell Counter (Invitrogen).

### RNAseq

RNA was extracted from undifferentiated iPS cells or iPS cell-derived cardiomyocytes using RNAeasy Micro Kit (QIAGEN, 74004). The extracted RNA was sequenced by strand-specific RNAseq. Forty to fifty million reads were obtained by 150 bp paired-end sequencing using DNBseq-400 (MGI Tech). Then, gene expression analysis such as heat map or GO enrichment analysis was performed using CLC workbench (QIAGEN).

### Cardiomyocyte differentiation from iPS cells and purification thereof

Basically, a technique of modulating Wnt signaling with small molecules was employed (11). Concentrations of CHIR99021 (R&D system) used for inducing differentiation were 6 µM and 12 µM. Both concentrations induced differentiation of the diploid iPS cells, but only 6 µM CHIR99021 induced differentiation of the tetraploid iPS cells into cardiomyocytes.

Fifteen days after the start of differentiation induction, a technique of purifying the cardiomyocytes using lactate was used (12). The diploid iPS cell-derived cardiomyocytes could be purified by replacing the medium containing 4 mM lactate three times, but the tetraploid iPS cell-derived cardiomyocytes died after the third medium replacement. Therefore, replacement with the medium containing 4 mM lactate was performed only twice. As well as the above purification process, the iPS cell-derived cardiomyocytes 28 days after the induction of differentiation were subjected to processes including freezing, RNA extraction, and immunostaining with an actinin antibody (Merck#A7732, 1/1000 dilution) (under the same conditions as for the undifferentiation marker genes described above).

### Hepatocyte differentiation from iPS cells

Basically, in the same manner as in the previous study (13), iPS cells were induced to differentiate into hepatocytes through induction into endodermal cells by Activin A treatment in the presence of a GSK-3 inhibitor, induction into hepatic progenitor cells by addition of a fibroblast growth factor FGF and an osteogenic factor BMP, and induction of maturation with hepatocyte growth factor HGF. Differentiation was evaluated by immunostaining (described above) with an albumin antibody (R&D#MAB1455, 1/200 dilution), a HNF4α antibody (CST#3113S, 1/200 dilution), and an AFP antibody (Sigma#A8452, 1/500 dilution), and quantitative PCR (described below).

### Skeletal muscle differentiation from iPS cells

Basically, the same technique as in the previous study was used (15). In addition to this technique, a medium containing 10 µM SB431542 (Fujifilm Wako), a TGF-β receptor inhibitor, was used 7 days after the induction of differentiation to improve differentiation efficiency. Differentiation was evaluated by morphological observation and immunostaining (described above) with a myosin heavy chain antibody (R&D#MAB4470, 1/400 dilution).

### Mitochondrial staining and image analysis

To the medium (RPMI1640 with B27 Serum-Free Supplement) in 12-well plates culturing diploid and tetraploid iPS cell-derived cardiomyocytes, 500 ng/ml of Hoechst 33342 (Merck) and 50 nM MitoTracker Green FM (M7514) were added and incubated at 37°C for 10 minutes. The medium was removed, the resultant was washed with PBS and observed under a fluorescence microscope, and images were taken. Subsequently, the number of mitochondria-positive pixels and the number of nuclei were automatically counted by image analysis using FiJi application to determine the amount of mitochondria per nucleus.

### Analysis of mitochondrial DNA copy number by digital PCR

Genomic DNA and mitochondrial DNA were extracted from the diploid and tetraploid iPS cell-derived cardiomyocytes. The genomic DNA copy number and the mitochondrial DNA copy number were detected as the copy number of the RPP30 gene sequence and the copy number of the MT-ND4 gene sequence, respectively. The copy number ratio of MT-ND4 gene to RPP30 gene was quantified by the copy number variation assay function of Droplet digital PCR (ddPCR, Bio-Rad). RPP30 was detected using RPP30 FW primer: GATTTGGACCTGCGAGCG (SEQ ID NO: 4), RPP30 RV primer: GCGGCTGTCTCCACAAGT (SEQ ID NO: 5), and RPP30 HEX probe: CTGACCTGAAGGCTCT (SEQ ID NO: 6), while MT-ND4 was detected using MT-ND4 FW primer: CCCTCATGTTCATACACCTATCC (SEQ ID NO: 1), MT-ND4 RV primer: AGCCTCTGTTGTCAGATTCAC (SEQ ID NO: 2), and MT-ND4 FAM probe: CCCATTCTCCTCCTATCCCTCAACCC (SEQ ID NO: 3).

### Observation with transmission electron microscope

At our request, the Tokyo Metropolitan Institute of Medical Science's Laboratory of Molecular Pathology and Histology conducted an observation of the diploid and tetraploid iPS cell-derived cardiomyocytes with a transmission electron microscope, using JEM1400 transmission electron microscope from JEOL Ltd.

### Quantitative PCR

Total RNA was extracted from iPS cells using Sepasol-RNAI Super G (Nacalai Tesque, Inc.). Residual DNA was then digested with DNase I (RNase-Free) (NEB). cDNA was synthesized from the total RNA using ReverTra Ace (registered trademark) qPCR RT Kit (TOYOBO).

The synthesized cDNA was used to perform droplet digital PCR (ddPCR) as follows, essentially using the same technique as in the previous study (14). As the primers/probe set, TaqMan (registered trademark) Gene Expression Assays were used (GAPDH: Hs99999905_m1 (VIC), AFP: Hs01040598_m1 (FAM), HNF4α: Hs00230853_m1 (FAM), ALB: Hs00609411_m1 (FAM)).

The thermal cycle conditions were as follows. 1, 95°C, 10 minutes; 2, 94°C, 30 seconds; 3, 59°C, 1 minute; 2 and 3 were repeated 39 times; and 4, 98°C, 10 minutes. The fluorescence signal of each oil droplet was analyzed by selecting CNV2 in QX100 droplet reader (Bio-Rad) and using QuantaSoft (Bio-Rad).

### Formation of embryoid body (EB) and cerebral organoid

For both diploid and tetraploid iPS cells, STEMdiff Cerebral Organoid Kit (#08570, STEMCELL^{™} TECHNOLOGIES) was used in accordance with the recommended protocol.

### Results

Establishment of diploid iPS cells that express fluorescent protein and drug-resistant gene

To select the fused cells using drugs, a puromycin-resistant gene and a blasticidin-resistant gene were introduced, each using a lentivirus, into the diploid iPS cells to be fused. To also visualize the fused cells with fluorescence, WTC11 and HPS0076 were established while co-transfecting Venus gene or mRFP1 gene and the puromycin-resistant gene or the blasticidin-resistant gene into the cells, respectively, so as allow for selection with each drug alone.

### Fusion of iPS cells with each other and drug selection

First, an attempt was made to fuse WTC11 with each other. The cells were fused using HVJ-E, and the fused cells were sorted out as cells resistant to both puromycin and blasticidin. The efficiency of cell fusion was examined by varying the amount of HVJ-E from 1 to 10 µl, and the fused cell clones were found to be established most efficiently with 1 µl. Approximately 20 tetraploid iPS cells could be established by fusing 2.0 × 10⁵ diploid cells each. The same procedure for establishing fused cells was also performed for fusion using polyethylene glycol, which is used for hybridoma establishment, etc. However, no fused cell clones could be obtained.

### Properties of established tetraploid iPS cells

Microscopic observation of the morphology of the established tetraploid iPS cells revealed that they were larger than normal diploid iPS cells and were mononuclear (Figure 1A). Co-expression of Venus and mRFP1 was also confirmed (Figure 1B). The measurement of the amount of DNA in the cells by flow cytometry confirmed that the amount of DNA in the tetraploid iPS cells clearly doubled compared to the diploid iPS cells (Figure 1C). Karyotyping also confirmed that the tetraploid iPS cells retained fully doubled chromosomes (Figure 1D). Furthermore, CGH analysis showed that chromosome doubling occurred without any small insertions or deletions (Figure 1E). Thus, the chromosome doubling by iPS cell fusion was complete without genetic abnormalities.

### Comparison of size and division cycle between tetraploid and diploid iPS cells and examination of retention of multipotency

Furthermore, the diameters of the cells were measured. The diploid cells were found to have a diameter of about 16.9 µm, while the tetraploid cells were about 1.1 times larger than that, with a diameter of about 18.7 µm (Figure 1F). The growth rates of the cells were measured. The diploid cells were found to have a doubling time of about 21.7 hours, while the tetraploid cells were about 1.2 times slower, with a doubling time of about 26.6 hours (Figure 1G).

To examine whether the tetraploid iPS cells retained their undifferentiated nature, expressions of the undifferentiation markers SOX2, OCT4, and SSEA4 were first confirmed by immunofluorescent staining, where the expressions were found to be retained as in the diploid iPS cells (Figure 2A). Furthermore, when the tetraploid iPS cells were transplanted into the testis of a NOD-SCID mouse, a teratoma was formed in the same manner as with the diploid iPS cells, and the characteristics of the triploblastic tissue could be observed (Figure 2B). The formed teratoma cells also retained their tetraploid ploidy level (Figure 2C). Thus, the tetraploid iPS cells retained their undifferentiated nature and multipotency even after DNA doubling.

### Comparison of transcript profiles between tetraploid and diploid iPS cells

RNA was extracted from the undifferentiated tetraploid and diploid iPS cells to compare the gene expressions by RNAseq. As a result, the number of genes showing reduced expressions in the tetraploid iPS cells compared to the diploid iPS cells was larger than the number of genes showing increased expressions in the tetraploid iPS cells. Genes showing increased expressions in the tetraploids included FAM187A, RN7SK, and RNU1-1. On the other hand, genes showing reduced expressions in the tetraploids included TRIM4, ZNF558, and UNC5D as well as many metallothionein (MT) genes, especially those that play roles in oxidative stress and metal toxicity responses (Figure 2D). When GO enrichment analysis was used to predict how the cellular functions would be affected by the gene group whose expressions varied according to their ploidy level, oxidative stress and metal toxicity responses accounted for 9 out of the top 10 (Figure 2E).

### Fusion of diploid iPS cells derived from different individuals with each other

Using the conditions under which WTC11 successfully fused with each other, an attempt was also made to fuse WTC11, which expresses the puromycin-resistant gene and Venus gene, with HPS0076, which expresses the blasticidin-resistant gene and mRFP1 gene. When 1 µl of HVJ-E was used, six tetraploid iPS cells could be established by fusing 2.0 × 10⁵ cells each. Thus, it was possible to fuse iPS cells using the same cells with each other or cells derived from different individuals with each other. Similar experiment involving fusion of similar iPS cells derived from different individuals was performed using polyethylene glycol. However, unlike the case with HVJ-E, no fused iPS cell lines were obtained. The established cells expressed both Venus and mRFP1 (Figure 3A) and were also confirmed to be tetraploids by flow cytometry (Figure 3B).

Furthermore, SNP-CGH analysis showed that homozygous single-nucleotide polymorphisms (SNPs) that differ between WTC11 and HPS0076 cells were detected as heterozygous SNPs in the established tetraploid cells. This indicates that the established tetraploid cells were clearly generated from the fusion of two different cell types. Typical SNPs are presented as examples (Table 1). Table 1 shows examples of SNPs detected as heterozygous SNPs in the fused cells as opposed to homozygous SNPs that differ between WTC11 and HPS0076 iPS cells. The tetraploid iPS cells established by fusing the iPS cells derived from different individuals could also be induced to differentiate into cardiomyocytes by the differentiation protocol described below (Figure 3C).

**[Table 1]**

| Table 1. SNPs of tetraploid iPS cells obtained through fusion of diploid iPS cells derived from different individuals. | | | | | |
|---|---|---|---|---|---|
| **Cell Lines SNP ID** | **Chr** | **SNP Position** | **WTC11 Genotype** | **HPS0076 Genotype** | **4N iPSCs Genotype** |
| **rs4646059** | **chr1** | **15830059** | **CC** | **AA** | **AC** |
| **rs11693904** | **chr2** | **3903993** | **AA** | **GG** | **GA** |
| **rs1499214** | **chr3** | **1390985** | **GG** | **TT** | **TG** |
| **rs4697998** | **chr4** | **10274626** | **TT** | **GG** | **GT** |
| **rs4866694** | **chr5** | **2886593** | **CC** | **TT** | **TC** |
| **rs6597076** | **chr6** | **4524418** | **CC** | **GG** | **GC** |
| **rs1541549** | **chr7** | **7788142** | **TT** | **CC** | **CT** |
| **rs921519** | **chr8** | **637069** | **CC** | **GG** | **GC** |
| **rs10491868** | **chr9** | **1306232** | **GG** | **TT** | **TG** |
| **rs4077784** | **chr10** | **1902429** | **GG** | **AA** | **AG** |
| **rs1391610** | **chr11** | **5427455** | **CC** | **TT** | **TC** |
| **rs2084660** | **chr12** | **7380974** | **CC** | **GG** | **GC** |
| **rs1334954** | **chr13** | **19584477** | **TT** | **GG** | **GT** |
| **rs12586813** | **chr14** | **21244479** | **AA** | **GG** | **GA** |
| **rs7497530** | **chr15** | **23766426** | **GG** | **AA** | **AG** |
| **rs2516739** | **chr16** | **2097158** | **GG** | **AA** | **AG** |
| **rs230474** | **chr17** | **3268520** | **CC** | **TT** | **TC** |
| **rs1394891** | **chr18** | **1222138** | **GG** | **CC** | **CG** |
| **rs4806907** | **chr19** | **3093163** | **CC** | **TT** | **TC** |

### Generation of octoploid iPS cells

In the same manner as the transfection of the puromycin- and blasticidin-resistant genes into the diploid WTC11 iPS cells using lentiviruses, a neomycin- or hygromycin-resistant gene was transfected into the tetraploid WTC11 iPS cells and selected with each drug. An attempt was made to generate octoploid iPS cells by fusing the obtained tetraploid WTC11 having the neomycin-resistant gene with the tetraploid WTC11 having the hygromycin-resistant gene.

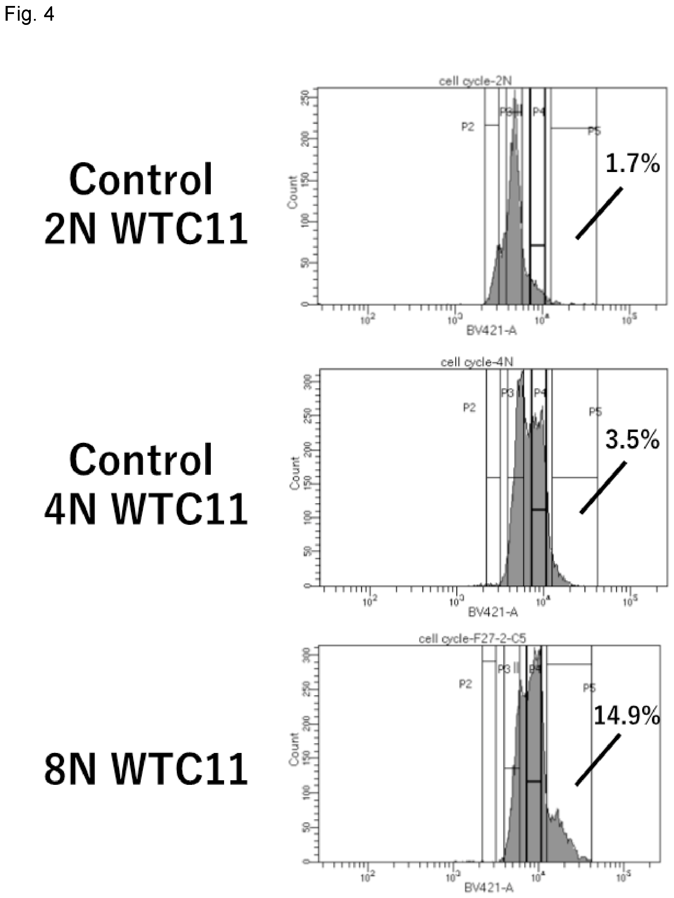

When 2.0 × 10⁵ cells each were fused with each other using 1 µl of HVJ-E, followed by selection with neomycin and hygromycin, the presence of octoploid iPS cells was observed by DNA quantification (Figure 4).

### Phenotype of cardiomyocytes derived from tetraploid iPS cells

The tetraploid iPS cells were allowed to differentiate into cardiomyocytes, and the phenotype was compared with that of diploid iPS cell-derived cardiomyocytes. Similar to the diploid cells, the tetraploid cells were able to differentiate into cardiomyocytes through modulation of Wnt signaling with small molecules (11). It was also possible to apply the technique of metabolically selecting only cardiomyocytes by adding lactate to the medium instead of glucose (12). However, although differentiation was successfully induced in the diploid iPS cells with either 6 µM or 12 µM CHIR99021, which was added at the beginning of the differentiation induction protocol to activate Wnt signaling, differentiation in the tetraploid iPS cells could be induced only with 6 µM CHIR99021 and not with 12 µM CHIR99021.

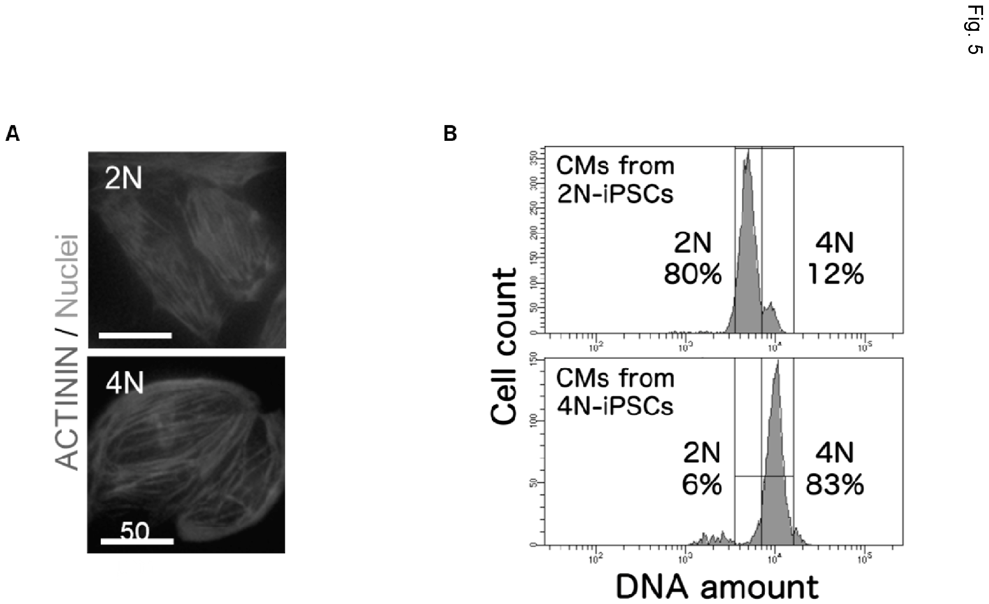

Moreover, in the process of metabolic selection of cardiomyocytes in a medium supplemented with lactic acid, the diploid iPS cell-derived cardiomyocytes could be selected by replacing the medium supplemented with lactic acid three times every two days. However, medium replacement could only be carried out up to twice for the tetraploid iPS cell-derived cardiomyocytes because the cardiomyocytes died after the third medium replacement. Thus, adjustments to the protocol were required to induce differentiation and select cardiomyocytes. Immunostaining confirmed that the obtained tetraploid iPS cell-derived cardiomyocytes, as well as the diploid iPS cell-derived cardiomyocytes, were positive for actinin, which constitutes a sarcomere. The size of the tetraploid iPS cell-derived cardiomyocytes were also found to be larger and as large as mature cardiomyocytes (Figure 5A).

When the amount of DNA in the obtained cardiomyocytes was quantified by flow cytometry, it was found that 80% of the diploid iPS cell-derived cardiomyocytes were diploids and only 12% were tetraploids, which does not reflect the conditions of an adult at all (Figure 5B). However, 83% of the tetraploid iPS cell-derived cardiomyocytes were tetraploids, reproducing the conditions of an adult more accurately. No octoploid cardiomyocytes were observed (Figure 5B).

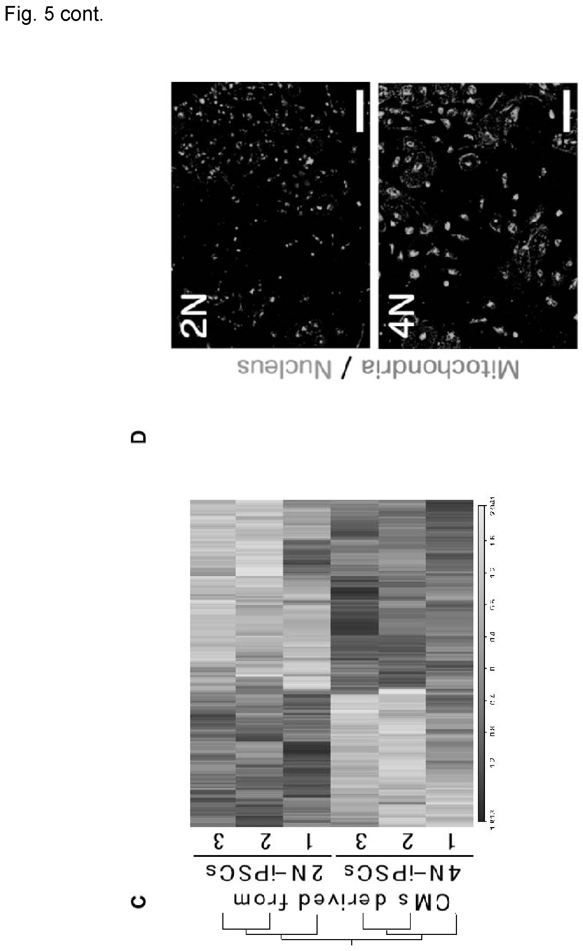

To compare the gene expression patterns, RNAseq was performed on the diploid and tetraploid iPS cell-derived cardiomyocytes. Three samples each were taken for comparison of gene expressions, which allowed clustering by groups of genes that are expressed at specific ploidy levels (Figure 5C). In the process of cardiomyocyte maturation, genes involved in cellular functions are known to switch isoforms such as from MYH6 to MYH7. Comparison of gene expressions in the cardiomyocytes using RNAseq revealed that this isoform switching occurred in the tetraploid iPS cell-derived cardiomyocytes compared to the diploid iPS cell-derived cardiomyocytes (Table 2). Table 2 shows comparison of the expressions of genes that underwent isoform switching between the tetraploid iPS cell-derived cardiomyocytes and the diploid iPS cell-derived cardiomyocytes.

### [Table 2]

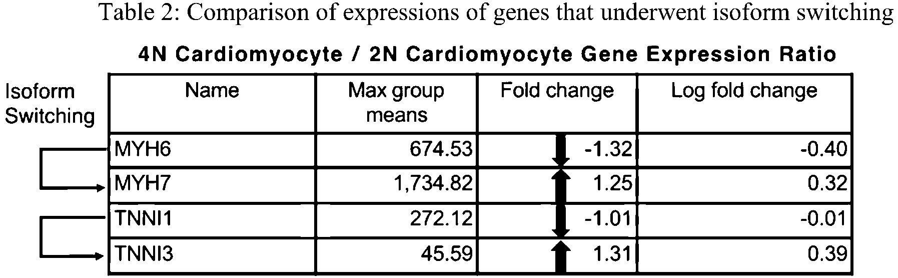

In addition, expression of KCNJ2, a ventricular myocyte ion channel whose expression increases with maturation, was found to be increased in the tetraploid iPS cell-derived cardiomyocytes. Conversely, expression of HCN4, an autonomic ion channel whose expression decreases with maturation, was decreased. Furthermore, the expressions of all genes encoded by mitochondrial DNA were found to be increased without exception (Table 3).

### [Table 3]

| Table 3. Comparison of mitochondrial gene expressions between diploid and tetraploid iPS cell-derived cardiomyocytes | | |
|---|---|---|
| MT-ATP6 | MT | 1.46 |
| MT-ATP8 | MT | 1.53 |
| MT-CO1 | MT | 1.15 |
| MT-CO2 | MT | 1.19 |
| MT-CO3 | MT | 1.32 |
| MT-CYB | MT | 1.41 |
| MT-ND1 | MT | 1.37 |
| MT-ND2 | MT | 1.55 |
| MT-ND3 | MT | 1.20 |
| MT-ND4 | MT | 1.22 |
| MT-ND4L | MT | 1.28 |
| MT-ND5 | MT | 1.21 |
| MT-ND6 | MT | 1.25 |
| MT-RNR1 | MT | NaN |
| MT-RNR2 | MT | NaN |

Meanwhile, when the expressions of these mitochondrial genes in the iPS cells in the undifferentiated state were analyzed by RNAseq, there was a mix of mitochondrial genes showing increased expression and those showing decreased expression between the diploid and tetraploid cells in the undifferentiated state (Table 4).

### [Table 4]

| Table 4. Comparison of mitochondrial gene expressions between diploid and tetraploid iPS cells | | |
|---|---|---|
| MT-ATP6 | MT | -1.10 |
| MT-ATP8 | MT | -1.12 |
| MT-CO1 | MT | -1.26 |
| MT-CO2 | MT | -1.01 |
| MT-CO3 | MT | -1.04 |
| MT-CYB | MT | -1.21 |
| MT-ND1 | MT | -1.29 |
| MT-ND2 | MT | -1.12 |
| MT-ND3 | MT | 1.06 |
| MT-ND4 | MT | -1.20 |
| MT-ND4L | MT | -1.04 |
| MT-ND5 | MT | -1.29 |
| MT-ND6 | MT | 1.21 |
| MT-RNR1 | MT | -1.13 |
| MT-RNR2 | MT | -1.14 |

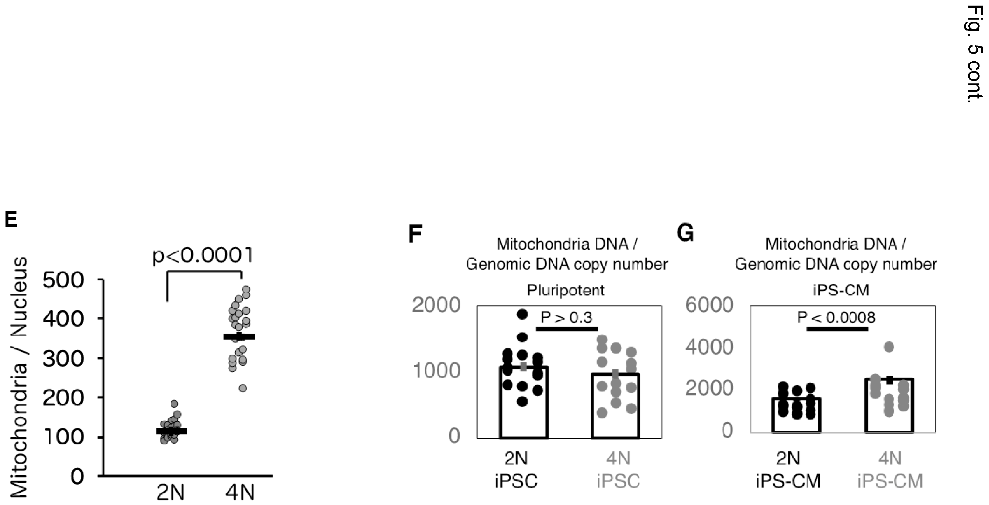

These results suggest that the amount of mitochondria increased and the ratio of mitochondrial DNA to nuclear DNA increased in the tetraploid iPS cell-derived cardiomyocytes, and that the increase in the amount of mitochondria was specific to cardiomyocyte differentiation. Therefore, the mitochondria were visualized for observation. An increase in mitochondria was observed in the tetraploid iPS cell-derived cardiomyocytes (Figures 5D and 5E). When the copy number ratio of mitochondrial DNA to nuclear DNA was quantified by digital PCR, no significant difference in the amount of mitochondria in the iPS cells in the undifferentiated state was observed between the diploid and tetraploid cells (Figure 5F), but an increase in mitochondria was observed in the tetraploid iPS cell-derived cardiomyocytes compared to the diploid iPS cell-derived cardiomyocytes (Figure 5G). Therefore, the increase in the amount of mitochondria was shown to occur specifically in cardiomyocytes.

Mitochondria play an extremely important role in the contractile function of cardiomyocytes, and the development of mitochondria is one of the indicators of cardiomyocyte maturation. Therefore, it can be said that the tetraploid iPS cell-derived cardiomyocytes reproduce the conditions of the adult heart more accurately than the conventional diploid iPS cell-derived cardiomyocytes.

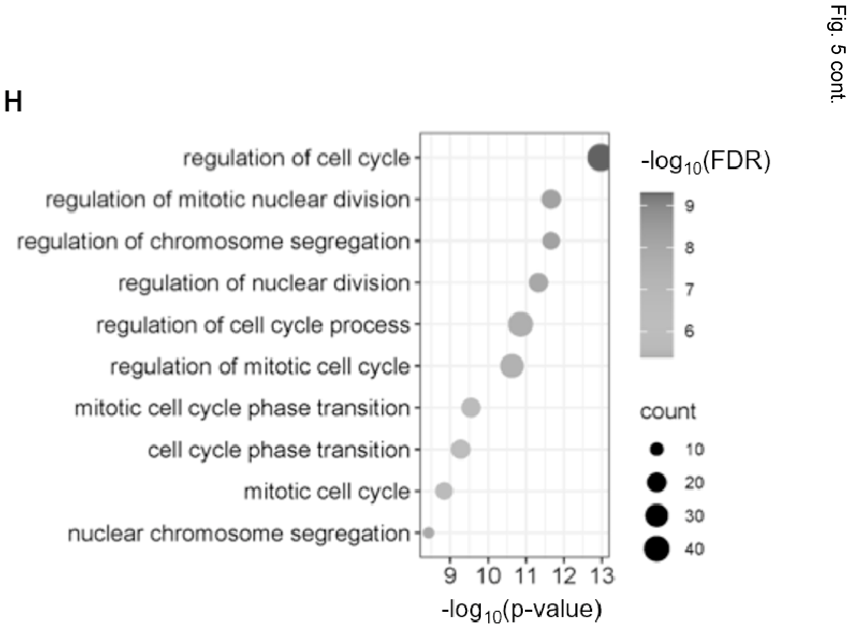

In addition, cellular functions involving a group of genes whose expressions varied in a ploidy level-specific manner were analyzed based on GO terms, and the top-ranked functions were all involved in the cell cycle (Figure 5H). All of them indicated that the cell cycle activity was significantly reduced in the tetraploid iPS cell-derived cardiomyocytes compared to the diploid iPS cell-derived cardiomyocytes. In the cases of cardiomyocytes *in vivo,* the fetal myocardium has proliferation potential, but this proliferation potential is lost as the myocardium matures. Thus, the tetraploid iPS cell-derived cardiomyocytes have characteristics that are more similar to those of mature cardiomyocytes.

Furthermore, the intracellular structures of the diploid and tetraploid iPS cell-derived cardiomyocytes were observed with a transmission electron microscope. Sarcomeres and mitochondria were observed in both intracellular structures (Figure 6).

### Phenotype of tetraploid iPS cell-derived hepatocytes

The tetraploid iPS cells were induced to differentiate into hepatocytes. Unlike the case of cardiomyocytes, hepatocyte differentiation was successfully induced using the same protocol as that used for inducing the differentiation of the diploid iPS cells. mRNA was extracted from the obtained hepatocytes to determine the expression levels of ALB, HNF4α, and AFP, which are markers for hepatocyte differentiation. The expression levels were higher in the tetraploid iPS cell-derived hepatocytes than in the diploid iPS cell-derived hepatocytes (Figure 7). As with the cardiomyocytes, polyploidized iPS cells could be used to increase the differentiation/maturity level.

### Induction of differentiation of tetraploid iPS cells into skeletal myocytes

The tetraploid iPS cells were induced to differentiate into skeletal myocytes. Similar to the hepatocyte differentiation, this differentiation was successfully induced using the same protocol as that used for inducing the differentiation of the diploid iPS cells. The induced skeletal myocytes were immunostained using myosin heavy chain as the marker, and their expression was confirmed in both diploids and tetraploids (Figure 8).

### Induction of formation of embryoid body (EB) and organoid from tetraploid iPS cells

An organoid formed from iPS cells is expected to reproduce a living tissue in three dimensions and serve as a more accurate model. Thus, in the same manner as in the case of diploid iPS cells, an attempt was made to form a cerebral organoid from the tetraploid iPS cells by differentiating the cells into an EB, and then further differentiating the EB into a cerebral organoid. As a result, similar to the diploid iPS cells, the tetraploid iPS cells formed an EB and further formed a cerebral organoid (Figures 9A and 9B). The cells of the formed organoid were also confirmed to have retained their ploidy level (Figure 9C).

### Discussion

There are reports of cases where pluripotent stem cells were fused with other cell types. For example, in the case where human fibroblasts were fused with ES cells using polyethylene glycol, the established cells retained expressions of the undifferentiation markers, indicating that the fibroblasts were reprogrammed by the fusion. Since the original purpose of this study was to examine whether the nuclei of fibroblasts could be reprogrammed by the fusion, the differentiation potential of the cells established by the fusion and the characteristics of the differentiated cells were not analyzed (8). There is another report indicating that fusion of iPS or ES cells with hematopoietic stem cells facilitates differentiation into mesendodermal cells. This report revealed that the established fused iPS cells can retain expressions of the undifferentiation marker genes, can differentiate into neurons, cardiomyocytes, and hepatocytes, and can form teratomas including triploblastic cells. In particular, differentiation into mesendodermal cells was promoted (9). It seems that the fusion of hematopoietic stem cells, which are mesodermal cells, with undifferentiated iPS cells has affected the differentiation potential.

On the other hand, the present invention is the first case where undifferentiated iPS cells are fused with each other, and it was found that this can promote differentiation into cardiomyocytes and hepatocytes, which are polyploidized particularly in normal living organisms. The promotion of cell differentiation allows for an accurate reproduction of cells in living organisms and allows improved analysis of disease models and their use in drug discovery research. This is also important in cell transplantation therapy.

For fusion between diploid cells, drug selection was performed using two drug-resistant genes, puromycin- and blasticidin-resistant genes, and octoploid cells could also be established by repeating the same procedure. By further repeating the procedure, hexadecaploids or higher polyploids can be generated. Some hepatocytes, skeletal myocytes, placentae, and megakaryocytes in living organisms are known to have very high ploidy levels (octoploids or higher polyploids), so it is effective to change the ploidy level of iPS cells according to the cell type of interest.

### REFERENCES

1. Thomson JA, Itskovitz-Eldor J, Shapiro SS, Waknitz MA, Swiergiel JJ, Marshall VS, et al. Embryonic stem cell lines derived from human blastocysts. Science. 1998 Nov 6; 282(5391): 1145-7.
2. Takahashi K, Tanabe K, Ohnuki M, Narita M, Ichisaka T, Tomoda K, et al. Induction of pluripotent stem cells from adult human fibroblasts by defined factors. Cell. 2007 Nov 30; 131(5): 861-72.
3. Ahmed RE, Anzai T, Chanthra N, Uosaki H. A Brief Review of Current Maturation Methods for Human Induced Pluripotent Stem Cells-Derived Cardiomyocytes. Front Cell Dev Biol. 2020 Mar 19; 8:178.
4. Derks W, Bergmann O. Polyploidy in cardiomyocytes: roadblock to heart regeneration? Circ Res. 2020 Feb 14; 126(4): 552-65.
5. Sladky VC, Eichin F, Reiberger T, Villunger A. Polyploidy control in hepatic health and disease. J Hepatol. 2021 Nov; 75(5): 1177-91.
6. Fang J, de Bruin A, Villunger A, Schiffelers R, Lei Z, Sluijter JPG. Cellular polyploidy in organ homeostasis and regeneration. Protein Cell. 2022 Dec 31;
7. Mitra S, Tomar PC. Hybridoma technology; advancements, clinical significance, and future aspects. J Genet Eng Biotechnol. 2021 Oct 18; 19(1): 159.
8. Cowan CA, Atienza J, Melton DA, Eggan K. Nuclear reprogramming of somatic cells after fusion with human embryonic stem cells. Science. 2005 Aug 26; 309(5739): 1369-73.
9. Qin J, Sontag S, Lin Q, Mitzka S, Leisten I, Schneider RK, et al. Cell fusion enhances mesendodermal differentiation of human induced pluripotent stem cells. Stem Cells Dev. 2014 Dec 1; 23(23): 2875-82.
10. Haim-Abadi G, Golan-Lev T, Koren A, Benvenisty N. Generation, genomic characterization, and differentiation of triploid human embryonic stem cells. Stem Cell Rep. 2023 Apr 17;
11. Miyaoka Y, Chan AH, Judge LM, Yoo J, Huang M, Nguyen TD, et al. Isolation of single-base genome-edited human iPS cells without antibiotic selection. Nat Methods. 2014 Mar; 11(3): 291-3.
12. Lian X, Hsiao C, Wilson G, Zhu K, Hazeltine LB, Azarin SM, et al. Robust cardiomyocyte differentiation from human pluripotent stem cells via temporal modulation of canonical Wnt signaling. Proc Natl Acad Sci USA. 2012 Jul 3; 109(27): E1848-57.
13. Tohyama S, Hattori F, Sano M, Hishiki T, Nagahata Y, Matsuura T, et al. Distinct metabolic flow enables large-scale purification of mouse and human pluripotent stem cell-derived cardiomyocytes. Cell Stem Cell. 2013 Jan 3; 12(1): 127-37.
14. Si-Tayeb K, Noto FK, Nagaoka M, Li J, Battle MA, Duris C, et al. Highly efficient generation of human hepatocyte-like cells from induced pluripotent stem cells. Hepatology. 2010 Jan; 51(1): 297-305.
15. Tanaka A, Woltjen K, Miyake K, Hotta A, Ikeya M, Yamamoto T, et al. Efficient and Reproducible Myogenic Differentiation from Human iPS Cells: Prospects for Modeling Miyoshi Myopathy In Vitro. PLOS ONE. 2013 Apr; 8(4): e61540.
16. Breckwoldt K, Letuffe-Brenière D, Mannhardt I, Schulze T, Ulmer B, et al. Differentiation of cardiomyocytes and generation of human engineered heart tissue. Nat Protoc. 2017 Jun; 12(6): 1177-1197.
17. Mandegar MA, Huebsch N, Frolov EB, Shin E, Truong A, Olvera MP, et al. CRISPR Interference Efficiently Induces Specific and Reversible Gene Silencing in Human iPSCs. Cell Stem Cell. 2016 Apr 7; 18(4): 541-53.

## Claims

1. A (N₁ + N₂)-ploid pluripotent stem cell comprising a fused cell of a N₁-ploid (wherein N₁ represents an integer from 2 to 16) pluripotent stem cell and a N₂-ploid (wherein N₂ represents an integer from 2 to 16) pluripotent stem cell.

2. The pluripotent stem cell according to claim 1, wherein N₁ and N₂ are 2 or 4.

3. The pluripotent stem cell according to claim 1, wherein the pluripotent stem cell is an iPS cell.

4. A method for producing a (N₁ + N₂)-ploid pluripotent stem cell, comprising a step of fusing a N₁-ploid (wherein N₁ represents an integer from 2 to 16) pluripotent stem cell and a N₂-ploid (wherein N₂ represents an integer from 2 to 16) pluripotent stem cell by culturing them in the presence of a virus.

5. The method according to claim 4, wherein N₁ and N₂ are 2 or 4.

6. The method according to claim 4, wherein the pluripotent stem cell is an iPS cell.

7. The method according to claim 4, wherein the virus is Sendai virus.

8. A method for producing a cardiomyocyte, comprising a step of culturing the pluripotent stem cell according to any one of claims 1-3 in the presence of a GSK-3 inhibitor.

9. A method for producing a hepatocyte, comprising the steps of: treating the pluripotent stem cell according to any one of claims 1-3 with Activin A in the presence of a GSK-3 inhibitor to induce it into an endodermal cell; treating the induced endodermal cell with a fibroblast growth factor and an osteogenic factor to induce it into a hepatic progenitor cell; and further treating the hepatic progenitor cell with a hepatocyte growth factor.

10. A method for producing a cerebral organoid, comprising the steps of: culturing the pluripotent stem cell according to any one of claims 1-3 in the presence of a fibroblast growth factor and a ROCK inhibitor to induce it into an embryoid body; culturing the embryoid body in a medium containing N2 supplement to induce it into a neuroepithelial cell; and further culturing it in a medium containing N2 supplement and B27 supplement.

11. A method for producing a cardiac organoid, comprising the following steps (a)-(d):
(a) a step of culturing the cell according to any one of claims 1-3 on a low attachment plate to induce it into an embryoid body;
(b) a step of treating the embryoid body obtained in step (a) with an GSK-3 inhibitor, an osteogenic factor, and Activin A to induce it into a mesoderm;
(c) a step of further treating the mesoderm induced in step (b) with Wnt-C59 to induce it into a cardiac mesoderm; and
(d) a step of treating the cardiac mesoderm induced in step (c) with a GSK-3 inhibitor.

12. A method for producing a hepatic organoid, comprising a step of culturing the pluripotent stem cell according to any one of claims 1-3 together with an iPS cell-derived endodermal cell, a vascular endothelial cell, and a mesenchymal stem cell to allow self-aggregation.

13. A method for producing a skeletal myocyte, comprising a step of culturing the pluripotent stem cell according to any one of claims 1-3 in the presence of a TGF-β receptor inhibitor.

14. A method for testing cardiotoxicity of a test substance, comprising the steps of: contacting the test substance with the pluripotent stem cell according to any one of claims 1-3, a cardiomyocyte generated by the method according to claim 8, or a cardiac organoid generated by the method according to claim 11; and evaluating the test substance as a cardiotoxic substance by using cell death, cell membrane potential, calcium transient, or reduced muscle contractility as an indicator.

15. A method for screening a drug that improves cardiomyocyte function, comprising the steps of: contacting a test substance with the pluripotent stem cell according to any one of claims 1-3, a cardiomyocyte generated by the method according to claim 8, or a cardiac organoid generated by the method according to claim 11; and selecting the test substance as a drug that improves cardiomyocyte function by using muscle contractility or beating rhythm as an indicator.

16. A method for testing hepatotoxicity of a test substance, comprising the steps of: contacting the test substance with the pluripotent stem cell according to any one of claims 1-3, a hepatocyte generated by the method according to claim 9, or a hepatic organoid generated by the method according to claim 12; and evaluating the test substance as a hepatotoxic substance by using cell death or mitochondrial toxicity as an indicator.

17. A method for screening a drug that improves hepatocyte function, comprising the steps of: contacting a test substance with the pluripotent stem cell according to any one of claims 1-3, a hepatocyte generated by the method according to claim 9, or a hepatic organoid generated by the method according to claim 12; and selecting the test substance as a drug that improves hepatocyte function by using drug-metabolizing ability as an indicator.

18. A method for screening a drug that improves skeletal myocyte function, comprising the steps of: contacting a test substance with the pluripotent stem cell according to any one of claims 1-3 or a skeletal myocyte generated by the method according to claim 13; and selecting the test substance as a drug that improves skeletal myocyte function by using contractility as an indicator.

19. A kit for testing cardiotoxicity of a test substance or for screening a drug that improves cardiomyocyte function, comprising the pluripotent stem cell according to any one of claims 1-3, a cardiomyocyte generated by the method according to claim 8, or a cardiac organoid generated by the method according to claim 11.

20. A kit for testing hepatotoxicity of a test substance or for screening a drug that improves hepatocyte function, comprising the pluripotent stem cell according to any one of claims 1-3, a hepatocyte generated by the method according to claim 9, or a hepatic organoid generated by the method according to claim 12.

21. A kit for screening a drug that improves skeletal myocyte function, comprising the pluripotent stem cell according to any one of claims 1-3 or a skeletal myocyte generated by the method according to claim 13.
